(19) Europäisches Patentamt
European Patent Office
Office européen des brevets

(11) Veröffentlichungsnummer: **0 195 201**
**B1**

(12) # EUROPÄISCHE PATENTSCHRIFT

(45) Veröffentlichungstag der Patentschrift: **23.05.90**

(21) Anmeldenummer: **86101048.6**

(22) Anmeldetag: **27.01.86**

(51) Int. Cl.[5]: **C 07 C 231/02,**
C 07 C 233/01, C 07 C 233/57,
C 07 C 233/64, C 07 C 327/20,
C 07 D 333/24,
C 07 D 333/28,
C 07 D 213/81, C 07 D 307/68

(54) **Verfahren zur Herstellung von N-Acyl-2,3-dehydroaminocarbonsäureestern.**

(30) Priorität: **11.03.85 DE 3508564**

(43) Veröffentlichungstag der Anmeldung:
**24.09.86 Patentblatt 86/39**

(45) Bekanntmachung des Hinweises auf die Patenterteilung:
**23.05.90 Patentblatt 90/21**

(84) Benannte Vertragsstaaten:
**AT BE CH DE FR GB IT LI LU NL SE**

(56) Entgegenhaltungen:
**EP-A-0 076 917**

**CHEMISCHE BERICHTE, Band 118, Nr. 12, Dezember 1985, Seiten 4869-4876; F. EFFENBERGER et al.: "Zersetzung von 2-Azidoketonen zu 2-(Acetylamino)-2-alken-1-onen unter Perrhenat-Katalyse"**

(73) Patentinhaber: **Degussa Aktiengesellschaft**
**Weissfrauenstrasse 9**
**D-6000 Frankfurt am Main 1 (DE)**

(72) Erfinder: **Effenberger, Franz, Prof. Dr. Dipl.-Chem.**
**Schatzweg 5**
**D-7000 Stuttgart (DE)**
Erfinder: **Kühlwein, Jürgen, Dipl.-Chem.**
**Sebastian-Bach-Strasse 4**
**D-7050 Waiblingen 7 (DE)**
Erfinder: **Drauz, Karlheinz, Dr. Dipl.-Chem.**
**Flurstrasse 5**
**D-6453 Freigericht 1 (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

EP 0 195 201 B1

Courier Press, Leamington Spa, England.

**Beschreibung**

Gegenstand der Erfindung ist ein Verfahren zur Herstellung von N-Acyl-2,3-dehydroaminocarbonsäureestern der allgemeinen Formel

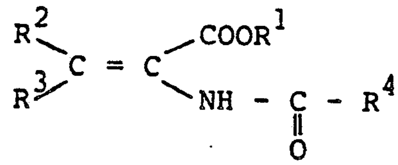 (I),

in der

$R^1$ einen geradkettigen oder verzweigten ($C_1$—$C_4$)-Alkylrest, einen Phenylrest oder einen Benzylrest,

$R^2$ Wasserstoff oder einen Methylrest,

$R^3$ Wasserstoff, einen geradkettigen oder verzweigten ($C_1$—$C_{16}$)-Alkylrest, einen ($C_3$—$C_8$)-Cycloalkylrest, einen ($C_1$—$C_6$)-Alkoxyrest, einen Phenoxyrest, einen ($C_1$—$C_6$)-Alkylmercaptorest, einen Phenylmercaptorest oder einen Methoxycarbonylmethylmercaptorest und

$R^4$ einen geradkettigen oder verzweigten unsubstituierten oder ein- oder mehrfach durch Halogen, eine Methoxygruppe oder eine Acetylthiogruppe substituierten ($C_1$—$C_{16}$)-Alkylrest oder ($C_2$—$C_{16}$)-Alkenylrest, einen unsubstituierten oder ein- oder mehrfach durch Halogen oder eine Methylgruppe substituierten ($C_3$—$C_8$)-Cycloalkylrest, einen unsubstituierten oder ein- oder mehrfach durch Halogen, eine Nitrogruppe, eine ($C_1$—$C_4$)-Alkyl- oder -Alkoxygruppe oder eine Trifluormethylgruppe substituierten Phenylrest, einen unsubstituierten oder ein- oder mehrfach durch Halogen substituierten Cinnamylrest, einen unsubstituierten oder ein- oder mehrfach durch Halogen substituierten Heteroarylrest, einen Aryl- oder Heteroarylmethylrest einen unsubstituierten oder ein- oder mehrfach durch Halogen, eine Nitrogruppe, eine ($C_1$—$C_4$)-Alkylgruppe oder eine Trifluormethylgruppe substituierten Phenoxymethylrest oder Phenylethylrest oder einen 2-(2',2'-Dichloroethenyl)-3,3-dimethylcyclopropylrest bedeuten.

Die N-Acyl-2,3-dehydroaminocarbonsäureester der allgemeinen Formel (I) sind als solche oder nach Verseifung der Esterfunktion wirksame Inhibitoren für die Dipeptidase [E.C.3.4.13.11.] und somit wertvolle Substanzen zur Erhaltung der Aktivität von β-Latamantibiotika, insbesondere aus der Thienamycingruppe. Sie können ferner auch als Zwischenprodukte für die Herstellung der entsprechenden optisch aktiven 2-Acylamino-carbonsäureester durch asymmetrische katalytische Hydrierung der prochiralen C=C-Doppelbindung dienen. Gegebenenfalls können die 2-Acylamino-carbonsäureester dann zu den entsprechenden optisch aktiven 2-Acylaminocarbonsäuren verseift werden.

Aus der DE—PS 31 40 227 ist es bereits bekannt, N-Acetyl-2,3-dehydroaminocarbonsäureester durch Umsetzung der entsprechenden 2-Azidocarbonsäureester mit einer Mischung aus Essigsäureanhydrid und Essigsäure in Gegenwart von Rhenium-(VII)-sulfid herzustellen. Dieses Verfahren erlaubt aber nur die Herstellung der N-Acetylverbindungen.

Das erfindungsgemäße Verfahren ist nun dadurch gekennzeichnet, daß man einen 2-Azidocarbonsäureester der allgemeinen Formel

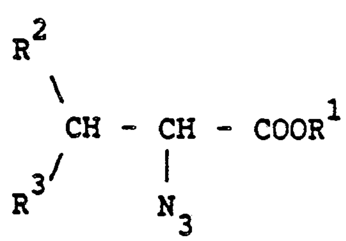 (II)

in der $R^1$, $R^2$ und $R^3$ die bereits angegebene Bedeutung haben, bei einer Temperatur zwischen 0 und 150°C in Gegenwart von Natrium-, Kalium- oder Ammoniumperrhenat oder eines substituierten Ammoniumperrhenats mit einem Carbonsäurehalogenid der allgemeinen Formel

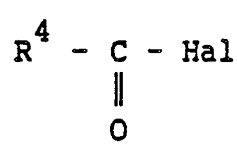 (III)

in der $R^4$ die bereits angegebene Bedeutung hat und Hal Chlor oder Brom bedeutet, zur Umsetzung bringt.

Auf diese Weise lassen sich alle N-Acyl-2,3-dehydroaminocarbonsäureester der allgemeinen Formel (I) in einem einstufigen Verfahren leicht und mit guter Ausbeute herstellen.

Das Perrhenat wird vorzugsweise in einer Menge zwischen 0,005 und 10 Molprozent, insbesondere

zwischen 0,1 und 3 Molprozent, bezogen auf die eingesetzte Menge an 2-Azidocarbonsäureester der allgemeinen Formel (II), angewandt.

Geeignete substituierte Ammoniumperrhenate sind beispielsweise Tetraethyl-, Tetrabutyl-, Triethylbenzyl- oder Tricaprylmethylammoniumperrhenat.

Unter Umständen ist es vorteilhaft, wenn man die Umsetzung des 2-Azidocarbonsäureesters der allgemeinen Formel (II) mit dem Carbonsäurehalogenid der allgemeinen Formel [III] in zusätzlicher Gegenwart eines Säureamids, eines Lactams oder eines substituierten Harnstoffs vornimmt. Diese als Aktivatoren dienenden Substanzen werden zweckmäßigerweise in mindestens etwa äquimolarer Menge zu dem eingesetzten Carbonsäurehalogenid der allgemeinen Formel (III) angewandt. Geeignete Säureamide sind beispielsweise Dimethylformamid, Dimethylacetamid oder Hexamethylphosphorsäuretriamid. Als Lactam wird das N-Methylpyrrolidon bevorzugt. Als substituierte Harnstoffe sind Tetramethylharnstoff und N,N'-Dimethyl-propylenharnstoff besonders gut verwendbar.

Die als Ausgangsmaterialien dienenden 2-Azidocarbonsäureester der allgemeinen Formel (II) können nach dem in Chem. Ber. *117*, Seiten 1497 bis 1512 (1984) beschriebenen Verfahren durch Umsetzung der entsprechenden 2-Chlor- oder 2-Brom-carbonsäureester mit einer wäßrigen Natriumazidlösung in Gegenwart eines Phasentransferkatalysators hergestellt werden. Beispiele für nach dem erfindungsgemäßen Verfahren umsetzbare 2-Azidocarbonsäureester der allgemeinen Formel (II) sind die Ester der 2-Azido-propionsäure, -buttersäure, -3-methylbuttersäure, -3-phenylpropionsäure, -pentansäure, -4-methylpentansäure, -3-cyclopentylpropionsäure, -3-cyclohexylpropionsäure, -hexansäure, -heptansäure, -octansäure, -nonansäure, -decansäure, -undecansäure, -dodecansäure, -tridecansäure, -tetradecansäure, -pentadecansäure, -hexadecansäure, -heptadecansäure, -octadecansäure, -nonadecansäure, -3-methylmercaptopropionsäure, -3-methoxycarbonylmethylmercaptopropionsäure, -3-phenylmercaptopropionsäure oder 3-Phenoxypropionsäure.

Die Carbonsäurehalogenide der allgemeinen Formel (III) können nach allgemein bekannten Verfahren aus den zugrundeliegenden Carbonsäuren mit Chlorierungsmitteln, wie Thionylchlorid, Phosphortrichlorid oder Phosgen bzw. Bromierungsmitteln, wie einem Gemisch aus rotem Phosphor und Brom oder Phosphortribromid, hergestellt werden. Als Carbonsäurehalogenide der allgemeinen Formel (III) werden vorzugsweise die Säurechloride eingesetzt. Die Carbonsäurehalogenide werden zweckmäßigerweise in einer Menge von 1,0 bis 1,5 Mol, vorzugsweise von 1,0 bis 1,1 Mol, pro Mol eingesetztem 2-Azidocarbonsäureester der allgemeinen Formel (II) angewandt.

Das erfindungsgemäße Verfahren wird vorzugsweise bei einer Temperatur zwischen 20 und 80°C durchgeführt.

Vorteilhafterweise wird die Umsetzung in einem inerten Lösungsmittel vorgenommen. Geeignete Lösungsmittel sind beispielsweise Carbonsäurealkylester, wie Essigsäureethylester, Essigsäurepropylester, Essigsäureisopropylester, Essigsäurebutylester, Propionsäureethylester; Acetonitril, Nitromethan, Tetrahydrofuran, Dioxan oder Dimethoxyethan. Anstelle eines zusätzlichen Lösungsmittels kann aber auch ein Überschuß der oben bereits genannten Säureamide, Lactame oder substituierten Harnstoffe eingesetzt werden.

Zur Vermeidung von Ausbeuteverlusten kann es zweckmäßig sein, die Umsetzung in Gegenwart einer geringen Menge eines Inhibitors für radikalische Polymerisationen, z.B. Hydrochinon oder Hydrochinonmonomethylether, vorzunehmen. Zweckmäßige Einsatzmengen für solche Inhibitoren liegen zwischen 0,001 und 10 Gewichtsprozent, insbesondere zwischen 0,1 und 3 Gewichtsprozent, bezogen auf den eingesetzten 2-Azidocarbonsäureester der allgemeinen Formel (II).

Bei gegen Säuren besonders empfindlichen 2-Azidocarbonsäureestern kann es vorteilhaft sein, die Umsetzung mit dem Carbonsäurehalogenid der allgemeinen Formel (III) in zusätzlicher Gegenwart eines Säureacceptors vorzunehmen, der zweckmäßigerweise in einer dem Carbonsäurehalogenid äquivalenten Menge eingesetzt wird. Geeignete Säureacceptoren sind insbesondere die Acetate oder Oxalate des Natriums oder Kaliums.

Das erfindungsgemäße Verfahren kann beispielsweise so durchgeführt werden, daß man den 2-Azidocarbonsäureester der allgemeinen Formel (II) in einem inerten Lösungsmittel vorlegt, das Perrhenat und gegebenenfalls den Aktivator und/oder Polymerisationsinhibitor zu der Lösung zugibt und schließlich das Carbonsäurehalogenid der allgemeinen Formel (III) zusetzt. Bei kleinen Ansätzen kann das Carbonsäurehalogenid auf einmal zugesetzt werden, bei größeren Ansätzen ist es empfehlenswerter, wenn es langsam, z.B. im Verlauf von 1 bis 3 Stunden zudosiert wird. Das Carbonsäurehalogenid kann in reiner Form oder als Lösung in dem verwendeten inerten Lösungsmittel zugegeben werden. Zweckmäßigerweise wird die Zugabegeschwindigkeit so gewählt, daß die Stickstoffentwicklung gut unter Kontrolle gehalten werden kann.

Wenn man das Carbonsäurehalogenid bei Raumtemperatur zugibt, kann die Reaktionstemperatur durch Wasserkühlung unter etwa 25°C gehalten werden. Arbeitet man ohne Außenkühlung, tritt nach kürzerer oder längerer Zeit eine Reaktionsexotherme auf. Dabei kann die Reaktionstemperatur kurzzeitig die Siedetemperatur des inerten Lösungsmittels erreichen. In jedem Fall wird das Reaktionsgemisch bis zur Beendigung der Gasentwicklung gerührt. Gegebenenfalls kann auch das Ende der Umsetzung IRspektroskopisch durch das Verschwinden der Azid-Bande festgestellt werden.

Nach beendeter Umsetzung wird das Lösungsmittel und gegebenenfalls der Aktivator, zweckmäßigerweise unter vermindertem Druck, beispielsweise mit einem Rotationsverdampfer, entfernt.

Der Rückstand wird in einem leichtflüchtigen Lösungsmittel, z.B. Essigsäureethylester, Essigsäureisopropylester oder Diethylether aufgenommen, mit einer wäßrigen Natriumhydrogencarbonatlösung ausgeschüttelt und anschließend mit Wasser nachgewaschen. Die über Natriumsulfat oder Magnesiumsulfat getrocknete organische Phase wird dann erneut unter vermindertem Druck, beispielsweise wieder in einem Rotationsverdampfer eingedampft. In vielen Fällen kann der nun verbleibende Rückstand durch Zugabe von n-Pentan zur Kristallisation gebracht werden. Dann werden die Kristalle abgesaugt und unter vermindertem Druck bei maximal 50°C getrocknet.

Handelt es sich bei dem Rückstand um ein nicht kristallisierendes Öl, kann eine weitere Reinigung durch eine Destillation im Hochvakuum, beispielsweise in einem Kugelrohr, versucht werden. In manchen Fällen kann es auch vorteilhaft sein, den Rückstand auf einer Kieselgelsäule durch Chromatographie zu reinigen. Dazu kann als Laufmittel ein Gemisch aus Essigsäureethylester und niedrigsiedendem Petrolether im Volumenverhältnis zwischen 1:1 und 1:9 verwendet werden. Nach Eindampfen des Eluats und Entfernung der letzten Lösungsmittelreste im Hochvakuum erhält man dann die analysenreinen N-Acyl-2,3-dehydroaminocarbonsäureester.

Die Erfindung wird durch die nachfolgenden Beispiele näher erläutert:

Beispiel 1

13,7 mg (0,05 mMol) Natriumperrhenat, 715,7 mg (5 mMol) 2-Azido-propansäureethylester und 383,8 mg (5,25 mMol) Dimethylformamid wurden in 5 ml Acetonitril gelöst. Dann wurden bei Raumtemperatur unter kräftigem Rühren 592,9 mg (5,25 mMol) Chloracetylchlorid zugegeben. Die Umsetzung verlief unter gleichmäßiger Stickstoffentwicklung innerhalb von 5 bis 6 Stunden. Nach beendeter Gasentwicklung wurde das Acetonitril unter vermindertem Druck entfernt. Der Rückstand wurde in Essigsäureethylester aufgenommen, mit 10,5 ml 0,5 molarer Natriumhydrogencarbonat-Lösung ausgeschüttelt, die abgetrennte organische Phase nochmals mit Wasser gewaschen und über Magnesiumsulfat getrocknet. Nach Entfernung des Trockenmittels wurde die eingeengte organische Phase mit einem Gemisch aus niedrig siedendem Petrolether und Essigsäureethylester im Volumenverhältnis 9:1 als Laufmittel über Kieselgel chromatographiert. Nach dem Eindampfen des Eluats hinterblieben 581,5 mg (60,7% der Theorie) analysenreiner 2-Chlormethylcarboxamido-propensäureethylester.

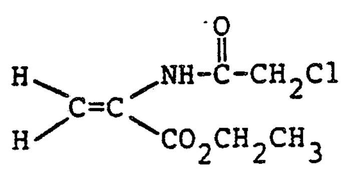

$C_7H_{10}ClNO_3$(191,614)

| | %C | %H | %N | %Cl |
|---|---|---|---|---|
| Berechnet: | 43,88 | 5,26 | 7,31 | 18,50 |
| Gefunden: | 43,99 | 5,40 | 7,02 | 18,46 |

Schmelzpunkt: 63°C

$^1H$—NMR($CDCl_3$): δ =
8,9 (s, 1H) NH;
6,64 (s, 1H) H—CH=;
6,0 (d, 1H) H—CH=;
4,37 (q, 2H) $OCH_2$—;
4,18 (s, 2H)—$CH_2$—Cl;
1,38 ppm (t, 3H)—$CH_8$.

IR (KBr)$\nu_{NH}$ = 3282 $cm^{-1}$,
$\nu_{CO}$ = 1716 $cm^{-1}$, 1679 $cm^{-1}$.

Beispiel 2

13,4 mg (0,05 mMol) Ammoniumperrhenat, 715,7 mg (5 mMol) 2-Azido-butansäuremethylester und 383,8 mg (5,25 mMol) Dimethylformamid wurden in 5 ml Acetonitril gelöst. Dann wurden bei Raumtemperatur unter kräftigem Rühren 592,9 mg (5,25 mMol) Chloracetylchlorid zugegeben. Das Reaktionsgemisch wurde 9 Stunden bei Raumtemperatur gerührt und anschließend analog Beispiel 1

aufgearbeitet. Es ergaben sich 845,6 mg (88,3 % der Theorie) analysenreiner 2-Chlormethylcarboxamido-2-butensäuremethylester.

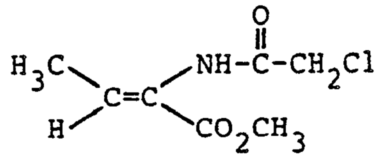

$C_7H_{10}ClNO_3$(191,614)

| | %C | %H | %N | %Cl |
|---|---|---|---|---|
| Berechnet: | 43,88 | 5,26 | 7,31 | 18,50 |
| Gefunden: | 43,75 | 5,23 | 7,19 | 18,37 |

Schmelzpunkt: 58,5 bis 59°C
$^1H$—NMR ($CDCl_3$): δ =
8,05 (s, 1H) NH;
6,95 (q, 1H) H—C($CH_3$)=;
4,19 (s, 2H) $CH_2Cl$;
3,80 (s, 3H) $OCH_3$;
1,87 ppm (d, 3H) $CH_2$—CH=.
IR (KBr)$\nu_{NH}$ = 3260 $cm^{-1}$,
$\nu_{CO}$ = 1727 $cm^{-1}$, 1676 $cm^{-1}$.

Beispiel 3

134 mg (0,5 mMol) Ammoniumperrhenat, 9,25 g (50 mMol) 2-Azido-butansäure-n-butylester und 3,85 g (52,6 mMol) Dimethylformamid wurden in 50 ml Acetonitril gelöst. Dann wurden bei Raumtemperatur unter kräftigem Rühren 5,93 g (52,5 mMol) Chloracetylchlorid zugegeben. Der Ansatz reagierte innerhalb 30 Minuten exotherm ab.

Nach beendeter Gasentwicklung wurde das Acetonitril unter vermindertem Druck entfernt. Der Rückstand wurde in Essigsäureethylester aufgenommen, mit 100 ml 0,5 molarer Natriumhydrogencarbonat-Lösung ausgeschüttelt, die abgetrennte organische Phase nochmals mit Wasser gewaschen und über Natriumsulfat getrocknet. Nach Entfernung des Trockenmittels wurde die eingeengte organische Phase mit n-Pentan zur Kristallisation gebracht, abgesaugt und getrocknet.

Es ergaben sich 9,8 g (83,9 % der Theorie) analysenreiner 2-Chlormethylcarboxamido-2-butensäure-n-butylester.

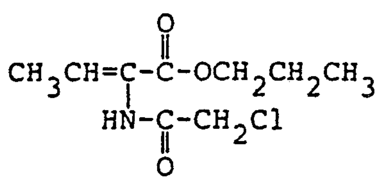

$C_{10}H_{16}NO_3Cl$(233,69)

| | %C | %H | %N | %Cl |
|---|---|---|---|---|
| Berechnet: | 51,40 | 6,90 | 5,99 | 15,17 |
| Gefunden: | 51,36 | 7,01 | 5,98 | 15,20 |

Schmelzpunkt: 53,5°C
$^1H$—NMR ($CDCl_3$/TMS): δ =
7,85 (s breit, 1H) NH;
6,8 (q, 1H) —CH=;
4,15 (t, 2H) —$COOCH_2$—;
4,1 (s, 2H) —$CH_2Cl$;
1,8 (d, 3H) $CH_3$—CH=;
0,6—2,0 ppm (m, 7H) —$CH_2$—$CH_2$—$CH_3$.

Beispiel 4

134 mg (0,5 mMol) Ammoniumperrhenat, 9,25 g (50 mMol) 2-Azido-butansäure-n-butylester und 3,85 g (52,6 mMol) Dimethylformamid wurden in 50 ml Acetonitril gelöst. Dann wurden bei Raumtemperatur unter kräftigem Rühren 7,75 g (52,5 mMol) Dichloracetylchlorid zugegeben. Die Exotherme setzte sofort ein und die Umsetzung war innerhalb 20 Minuten beendet.

Die Aufarbeitung erfolgte analog Beispiel 3. Es ergaben sich 11,2 g (83,5% der Theorie) analysenreiner 2-Dichlormethylcarboxamido-2-butensäure-n-butylester.

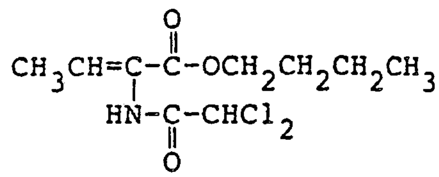

$C_{10}H_{15}NO_3Cl_2$(268,14)

| | %C | %H | %N | %Cl |
|---|---|---|---|---|
| Berechnet: | 44,79 | 5,64 | 5,22 | 26,44 |
| Gefunden: | 44,75 | 5,81 | 5,20 | 25,16 |

Schmelzpunkt: 56 bis 58,5°C
$^1H$—NMR ($CDCl_3$/TMS): δ =
7,85 (s breit, 1H) NH;
6,85 (q, 1H) —CH=;
5,95 (s, 1H) —$CHCl_2$;
4,1 (t, 2H) —$COOCH_2$—;
1,75 (d, 3H) $CH_3$—CH=;
0,6—1,85 ppm (m, 7H) —$CH_2$—$CH_2$—$CH_3$.

Beispiel 5

134 mg (0,5 mMol) Ammoniumperrhenat, 9,25 g (50 mMol) 2-Azido-butansäure-n-butylester, 50 mg (0,45 mMol) Hydrochinon und 3,85 g (52,6 mMol) Dimethylformamid wurden in 50 ml Acetonitril gelöst. Dann wurden bei Raumtemperatur unter kräftigem Rühren 14,4 g (52,3 mMol) Hexadecansäurechlorid zugegeben. Der Ansatz reagierte innerhalb 1,5 Stunden exotherm ab. Nach dem Abkühlen fiel das Rohprodukt aus, wurde abgesaugt und mit Wasser Chlorid-frei gewaschen. Es ergaben sich 19,5 g (98,5% der Theorie) analysenreiner 2-(Pentadecylcarbonylamino)-2-butensäure-n-butylester.

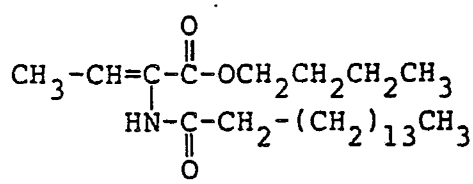

$C_{24}H_{45}NO_3$(395,63)

| | %C | %H | %N |
|---|---|---|---|
| Berechnet: | 72,86 | 11,46 | 3,54 |
| Gefunden: | 72,86 | 11,76 | 3,18 |

Schmelzpunkt: 66 bis 69°C
$^1H$—NMR ($CDCl_3$/TMS): δ =
6,9 (s breit, 1H) NH;
6,75 (q, 1H) —CH=;
4,1 (t, 2H) —$COOCH_2$;
2,25 (t, 2H) —$COCH_2$—;
1,75 (d, 3H) $CH_3$—CH=;
0,55—1,65 ppm (m, 36H) —$(CH_2)_{13}CH_3$; —$(CH_2)_3$—$CH_3$.

Beispiel 6

134 mg (0,5 mMol) Ammoniumperrhenat, 7,85 g (50 mMol) 2-Azido-pentansäuremethylester, 50 mg (0,45 mMol) Hydrochinon und 3,85 g (52,6 mMol) Dimethylformamid wurden in 50 ml Acetonitril gelöst. Dann wurden bei Raumtemperatur unter kräftigem Rühren 10,6 g (52,3 mMol) 10-Undecensäurechlorid zugegeben. Der Ansatz reagierte innerhalb 30 Minuten exotherm ab, er wurde anschließend analog Beispiel 3 aufgearbeitet.

Das so erhaltene Rohprodukt wurde im Hochvakuum (0,06 mbar) bei 200 bis 250°C Gerätetemperatur

in einem Kugelrohr destilliert. Es ergaben sich 11,5 g (77,8% der Theorie) analysenreiner 2-(Decen-9-yl-carbonylamino)-2-pentensäuremethylester.

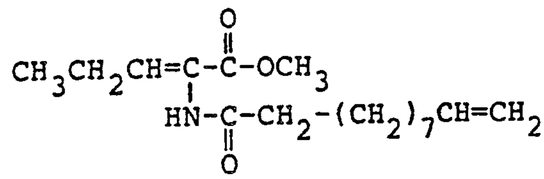

$C_{17}H_{29}NO_3$(295,42)

| | %C | %H | %N |
|---|---|---|---|
| Berechnet: | 69,12 | 9,89 | 4,74 |
| Gefunden: | 68,91 | 10,16 | 4,36 |

Schmelzpunkt: 34 bis 35°C
$^1$H—NMR (DMSO/TMS): δ =
9,1 (s, 1H) NH;
6,3 (t, 1H) —CH=CO;
5,4—6,05 (m, 1H) $—CH_2—CH=CH_2$;
4,6—5,2 (m, 2H) $—CH=CH_2$;
3,6 (s, 3H) $—COOCH_3$;
1,65—2,5 (m, 6H) $CH_3CH_2$; $—COCH_2$; $—CH_2=CH$;
0,7—1,75 ppm (m, 15H) $CH_3$; $—(CH_2)_6$.

Beispiel 7

146,6 mg (0,5 mMol) Kaliumperrhenat, 7,85 g (50 mMol) 2-Azido-pentansäuremethylester und 50 mg (0,45 mMol) Hydrochinon wurden bei 80°C in 25 ml Essigsäureethylester vorgelegt. Dann wurden innerhalb von 30 Minuten unter kräftigem Rühren 5,93 g (52,5 mMol) Chloracetylchlorid, gelöst in 25 ml Essigsäureethylester, zugetropft. Das Reaktionsgemisch wurde unter kräftigem Rühren insgesamt 2,5 Stunden auf 80°C gehalten und anschließend analog Beispiel 3 aufgearbeitet. Es ergaben sich 7,0 g (68,1% der Theorie) analysenreiner 2-Chlormethylcarboxamido-2-pentansäuremethylester.

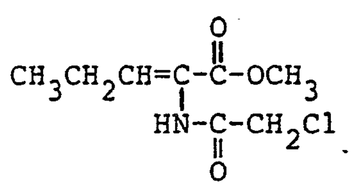

$C_8H_{12}Cl$(205,64)

| | %C | %H | %N | %Cl |
|---|---|---|---|---|
| Berechnet: | 46,72 | 5,84 | 6,81 | 17,24 |
| Gefunden: | 46,89 | 5,81 | 6,80 | 17,31 |

Schmelzpunkt: 62°C
$^1$H—NMR ($CDCl_3$/TMS): δ =
7,85 (s breit, 1H) NH;
6,75 (t, 1H) —CH=;
4,15 (s, 2H) $—CH_2Cl$;
3,8 (s, 3H) $COOCH_3$
1,7—2,65 (m, 2H) $—CH_2—$;
1,05 ppm (t, 3H) $CH_3—$.

Beispiel 8

138,5 mg (0,5 mMol) Natriumperrhenat, 7,85 g (50 mMol) 2-Azido-pentansäuremethylester und 50 mg (0,45 mMol) Hydrochinon wurden bei 80°C in 25 ml Essigsäureethylester vorgelegt. Dann wurden innerhalb von 30 Minuten unter kräftigem Rühren 7,75 g (52,5 mMol) Dichloracetylchlorid, gelöst in 25 ml Essigsäureethylester, zugetropft. Das Reaktionsgemisch wurde unter kräftigem Rühren insgesamt 3 Stunden auf 80°C gehalten und anschließend analog Beispiel 3 aufgearbeitet.

Es ergaben sich 7,5 g (62,5% der Theorie) analysenreiner 2-Dichlormethylcarboxamido-2-pentensäuremethylester.

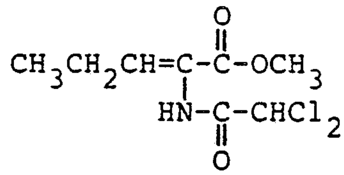

$C_8H_{11}NO_3Cl_2$(240,087)

| | %C | %H | %N | %Cl |
|---|---|---|---|---|
| Berechnet: | 40,02 | 4,62 | 5,83 | 29,53 |
| Gefunden: | 40,22 | 4,62 | 5,82 | 29,65 |

Schmelzpunkt: 85°C
$^1$H—NMR ($CDCl_3$/TMS): δ =
7,8 (s breit, 1H) NH;
68 (t, 1H) —CH=;
6,05 (s, 1H) $CHCl_2$;
3,85 (s, 3H) $COOCH_3$;
1,75—2,6 (m, 2H) —$CH_2$—;
1,1 ppm (t, 3H) $CH_3$—.

## Beispiel 9

134 mg (0,5 mMol) Ammoniumperrhenat, 9,26 g (50 mMol) 2-Azido-pentansäure-isopropylester und 50 mg (0,45 mMol) Hydrochinon wurden bei 80°C in 25 ml Essigsäureethylester vorgelegt. Dann wurden innerhalb von 45 Minuten Unter kräftigem Rühren 5,93 g (52,5 mMol) Chloracetylchlorid, gelöst in 25 ml Essigsäureethylester, zugetropft. Das Reaktionsgemisch wurde unter kräftigem Rühren insgesamt 4 Stunden auf 80°C gehalten und anschließend analog Beispiel 3 aufgearbeitet.

Es ergaben sich 7,3 g (62,5% der Theorie) analysenreiner 2-Chlormethylcarboxamido-2-pentensäureisopropylester.

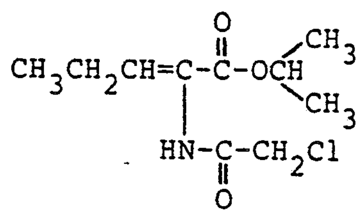

$C_{10}H_{16}NO_3Cl$(233,695)

| | %C | %H | %N | %Cl |
|---|---|---|---|---|
| Berechnet: | 51,39 | 6,90 | 5,99 | 15,17 |
| Gefunden: | 51,87 | 6,68 | 5,94 | 15,24 |

Schmelzpunkt: 96°C
$^1$H—NMR ($CDCl_3$/TMS): δ =
7,85 (s breit, 1H) NH;
6,65 (t, 1H) —CH=;
4,7—5,65 (m, 1H) —COOCH;
4,15 (s, 1H) —$CHCl_2$;
1,65—2,65 (m, 2H) —$CH_2$—;
1,3 (d, 6H), C—$(CH_3)_2$;
1,05 ppm (t, 3H) $CH_3$—.

## Beispiel 10

134 mg (0,5 mMol) Ammoniumperrhenat, 9,26 g (50 mMol) 2-Azido-pentansäureisopropylester und 50 mg (0,45 mMol) Hydrochinon wurden bei 80°C in 25 ml Essigsäureethylester vorgelegt. Dann wurden innerhalb von 70 Minuten unter kräftigem Rühren 7,75 g (52,5 mMol) Dichloracetylchlorid, gelöst ind 25 ml Essigsäureethylester, zugetropft. Das Reaktionsgemisch wurde unter kräftigem Rühren insgesamt 5 Stunden auf 80°C gehalten und anschließend analog Beispiel 3 aufgearbeitet.

Es ergaben sich 10,6 g (79% der Theorie) analysenreiner 2-Dichlormethylcarboxamido-2-pentensäureisopropylester.

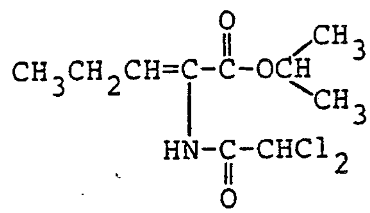

$C_{10}H_{15}NO_3Cl_2$(268,14)

| | %C | %H | %N | %Cl |
|---|---|---|---|---|
| Berechnet: | 44,79 | 5,63 | 5,22 | 26,44 |
| Gefunden: | 45,25 | 5,50 | 5,20 | 26,38 |

Schmelzpunkt: 110°C
$^1H$—NMR ($CDCl_3$/TMS): δ =
7,85 (s breit, 1H) NH;
6,7 (t, 1H) —CH=;
6,0 (s, 1H) $CHCl_2$;
4,75—5,4 (m, 1H) COOCH;
1,8—2,5 (m, 2H) —$CH_2$—;
1,3 (d, 6H) —C—$(CH_3)_2$;
1,05 (t, 3H) $CH_3$—.

## Beispiel 11

134 mg (0,5 mMol) Ammoniumperrhenat, 7,85 g (50 mMol) 2-Azido-pentansäuremethylester und 50 mg (0,45 mMol) Hydrochinon wurden bei 80°C in 25 ml Essigsäureethylester vorgelegt. Dann wurden innerhalb von 30 Minuten 9,3 g (52,6 mMol) 2,6-Difluorbenzoylchlorid, gelöst in 25 ml Essigsäureethylester, zugetropft. Das Reaktionsgemisch wurde unter kräftigem Rühren insgesamt 10 Stunden auf 80°C gehalten und anschließend analog Beispiel 3 aufgearbeitet. Es ergaben sich 9,75 g (72,4% der Theorie) analysenreiner 2-[(2′,6′-Difluorphenyl)-carbonylamino]-2-pentensäuremethylester.

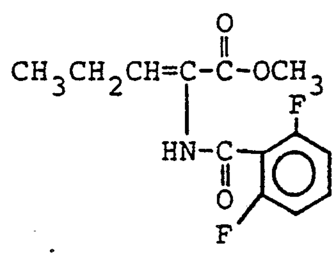

$C_{12}H_{13}O_3NF_2$(269,25)

| | %C | %H | %N |
|---|---|---|---|
| Berechnet: | 57,19 | 4,86 | 5,20 |
| Gefunden: | 58,12 | 4,58 | 5,13 |

Schmelzpunkt: 138,5°C
$^1H$—NMR ($CDCl_3$/TMS): δ =
6,4—7,85 (m, 5H) aromat.—CH; —CH=; NH;
3,8 (s, 3H) $COOCH_3$;
1,85—2,65 (m, 2H) —$CH_2$—;
1,1 ppm (t, 3H) $CH_3$—.

## Beispiel 12

146,6 mg (0,5 mMol) Kaliumperrhenat, 9,26 g (50 mMol) 2-Azido-pentansäureisopropylester und 50 mg (0,45 mMol) Hydrochinon wurden bei 80°C in 25 ml Essigsäureethylester vorgelegt. Dann wurden innerhalb von 30 Minuten 9,3 g (52,6 mMol) 2,6-Difluorbenzoylchlorid, gelöst in 25 ml Essigsäureethylester, zugetropft. Das Reaktionsgemisch wurde unter kräftigem Rühren insgesamt 10 Stunden auf 80°C gehalten

und anschließend analog Beispiel 3 aufgearbeitet. Es ergaben sich 10,9 g (73,3% der Theorie) analysenreiner 2-[(2',6'-Difluorphenyl)-carbonylamino]-2-pentensäureisopropylester.

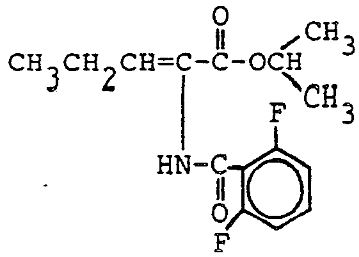

$C_{15}H_{17}O_3NF_2$(297,30)

| | %C | %H | %N |
|---|---|---|---|
| Berechnet: | 60,60 | 5,76 | 4,71 |
| Gefunden: | 61,14 | 5,50 | 4,66 |

Schmelzpunkt: 91°C

$^1H$—NMR ($CDCl_3$/TMS): δ =
6,45—7,9 (m, 5H) aromat.—CH; —CH=; NH;
4,7—5,4 (m, 1H) COOCH;
1,75—2,65 (m, 2H) —$CH_2$—;
1,3 (d, 6H) C—$(CH_3)_2$;
1,1 ppm (t, 3H) $CH_3$.

Beispiel 13

536 mg (19,9 mMol) Ammoniumperrhenat, 31,4 g (0,2 mMol) 2-Azido-pentansäuremethylester und 15,4 g (0,21 mMol) Dimethylformamid wurden in 150 ml Acetonitril gelöst. Dann wurden bei Raumtemperatur unter kräftigem Rühren 25,3 g (0,21 mMol) 3-Methyl-butansäurechlorid zugegeben. Der Ansatz reagierte innerhalb 45 Minuten exotherm ab. Die Aufarbeitung erfolgte analog Beispiel 3. Es ergaben sich 30,2 g (70,8% der Theorie) analysenreiner 2-[(2'-Methylpropyl)-carbonylamino]-2-pentensäuremethylester.

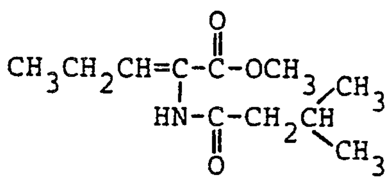

$C_{11}H_{19}NO_3$(213,28)

| | %C | %H | %N |
|---|---|---|---|
| Berechnet: | 61,95 | 8,98 | 6,57 |
| Gefunden: | 61,84 | 9,01 | 6,51 |

Schmelzpunkt: 45 bis 47°C

$^1H$—NMR ($CDCl_3$/TMS): δ =
7,05 (s breit, 1H) NH;
6,65 (t, 1H) —CH=;
3,75 (s, 3H) —$COOCH_3$;
1,7—2,6 (m, 5H) —$CH_2$—C=; C(=O)—$CH_2$—CH—;
1,15 (s, 3H) $CH_3$—$CH_3$;
1,0 ppm (d, 6H) C($CH_3$)($CH_3$)

Beispiel 14

134 mg (0,5 mMol) Ammoniumperrhenat, 7,85 g (50 mMol) 2-Azido-pentansäuremethylester, 3,85 g (52,6 mMol) Dimethylformamid und 50 mg (0,45 mMol) Hydrochinon wurden in 50 ml Acetonitril gelöst. Dann wurden bei Raumtemperatur unter kräftigem Rühren 8,5 g (52,3 mMol) 2-Ethyl-hexanoylchlorid

zugegeben. Das Reaktionsgemisch wurde 120 Stunden gerührt, wobei die Reaktionsexotherme eine maximale Temperatur von 80°C erreichte.

Die Aufarbeitung erfolgte analog Beispiel 3. Das so erhaltene Rohprodukt wurde im Hochvakuum (0,06 mbar) bei 180 bis 210°C Gerätetemperatur im Kugelrohr destilliert. Es ergaben sich 7,0 g (54,8% der Theorie) 2-[(1′-Ethylpentyl)-carbonylamino]-2-pentensäuremethylester.

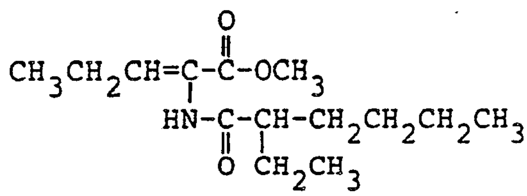

$C_{10}H_{25}NO_3$(255,36)

| | %C | %H | %N |
|---|---|---|---|
| Berechnet: | 65,85 | 9,87 | 5,48 |
| Gefunden: | 65,11 | 10,31 | 5,52 |

$^1$H—NMR ($CDCl_3$/TMS): δ =
6,85 (s breit, 1H) NH;
6,55 (t, 1H) —CH=;
3,7 (s, 3H) —$COOCH_3$;
0,6—2,45 ppm (m, 20H) $CH_3CH_2$—C=; —CH—$(CH_2)_3$—$CH_3$.
|
$CH_2CH_3$

Beispiel 15

134 mg (0,5 mMol) Ammoniumperrhenat, 7,85 g (50 mMol) 2-Azido-pentansäuremethylester, 3,85 g (52,6 mMol) Dimethylformamid und 50 mg (0,45 mMol) Hydrochinon wurden in 50 ml Acetonitril gelöst. Dann wurden bei Raumtemperatur unter kräftigem Rühren 7,8 g (52,5 mMol) 2-Methyl-2-Ethyl-butansäurechlorid zugegeben. Das Reaktionsgemisch wurde 120 Stunden gerührt, wobei die Reaktionsexotherme eine maximale Temperatur von 80°C erreichte.

Die Aufarbeitung erfolgte analog Beispiel 3. Das so erhaltene ölige Rohprodukt wurde im Hochvakuum (0,06 mbar) bei 100 bis 150°C Gerätetemperatur im Kugelrohr destilliert. Es ergaben sich 6,4 g (53% der Theorie) 2-[(1′-Methyl-1′-ethyl-propyl)-carbonylamino]-2-pentensäuremethylester.

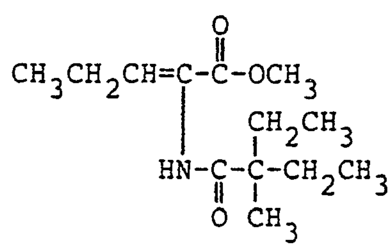

$C_{13}H_{23}NO_3$(241,33)

| | %C | %H | %N |
|---|---|---|---|
| Berechnet: | 64,70 | 9,61 | 5,80 |
| Gefunden: | 63,70 | 10,16 | 6,25 |

$^1$H—NMR ($CDCl_3$/TMS): δ =
7,15 (s breit, 1H) NH;
6,65 (t, 1H) —CH=;
3,75 (s, 3H) —$COOCH_3$;
0,65—2,35 ppm (m, 18H) $CH_3CH_2$—C=; —C—$(CH_2CH_3)_2$.
|
$CH_3$

Beispiel 16

134 mg (0,5 mMol) Ammoniumperrhenat, 7,85 g (50 mMol) 2-Azido-pentansäuremethylester, 50 mg (0,45 mMol) Hydrochinon und 3,85 g (52,6 mMol) Dimethylformamid wurden in 50 ml Essigsäureethylester gelöst. Dann wurden bei Raumtemperatur unter kräftigem Rühren 8,5 g (52,9 mMol) Thiophen-2-essigsäurechlorid zugetropft. Der Ansatz reagierte innerhalb 30 Minuten exotherm ab.

Nach beendeter Gasentwicklung wurde das Reaktionsgemisch mit 50 ml Essigsäureethylester

verdünnt, mit 100 ml 0,5 molarer Natriumhydrogencarbonat-Lösung ausgeschüttelt, die abgetrennte organische Phase nochmals mit Wasser gewaschen und über Natriumsulfat getrocknet. Nach Entfernung des Trockenmittels wurde die eingeengte organische Phase mit n-Pentan zur Kristallisation gebracht.

Es ergaben sich 12,1 g (95,5% der Theorie) analysenreiner 2-(Thenyl-carbonylamino)-2-pentensäuremethylester.

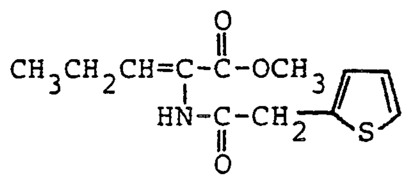

$C_{12}H_{15}NO_3S$(253,26)

| | %C | %H | %N | %S |
|---|---|---|---|---|
| Berechnet: | 56,91 | 5,97 | 5,53 | 12,63 |
| Gefunden: | 56,80 | 5,86 | 5,66 | 12,63 |

Schmelzpunkt: 67 bis 70°C
$^1H$—NMR ($CDCl_3$/TMS): δ =

6,85—7,5 (m, 4H) NH; 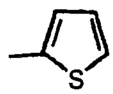 ;
6,65 (t, 1H) CH=;
3,9 (s, 2H) $COCH_2$;
3,75 (s, 3H) $COOCH_3$;
1,65—2,55 (m, 2H) $—CH_2—$;
1,05 ppm (t, 3H) $CH_3—$.

Beispiel 17

134 mg (0,5 mMol) Ammoniumperrhenat, 7,85 g (50 mMol) 2-Azido-pentansäuremethylester, 50 mg (0,45 mMol) Hydrochinon und 3,85 g (52,6 mMol) Dimethylformamid wurden analog Beispiel 16 mit 9,5 g (52,6 mMol) 3-Acetylthio-2-methylpropionsäurechlorid umgesetzt.

Nach Aufarbeitung analog Beispiel 16 ergaben sich 10,9 g (79,8% der Theorie) analysenreiner 2-[(1'-Methyl-2'-acetylthio-ethyl)-carboxamido]-2-pentensäuremethylester.

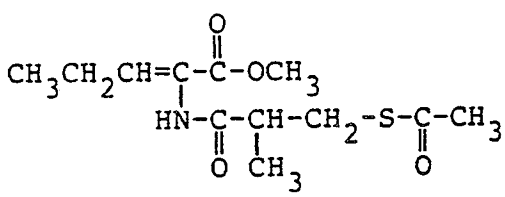 .

$C_{12}H_{19}NO_4S$(273,29)

| | %C | %H | %N | %S |
|---|---|---|---|---|
| Berechnet: | 52,74 | 7,01 | 5,12 | 11,71 |
| Gefunden: | 52,46 | 7,01 | 5,22 | 11,56 |

Schmelzpunkt: 56 bis 58°C
$^1H$—NMR ($CDCl_3$/TMS): δ =
7,55 (s breit, 1H) NH;
6,65 (t, 1H) CH=;
3,8 (s, 3H) $COOCH_3$;
3,1 (t, 2H) $S—CH_2—$;
2,4—2,9 (m, 1H) COCH;
2,35 (s, 3H) $COCH_3$;
1,8—2,4 (m, 2H) $—CH_2—$;
1,25 (d, 3H) $CH—CH_3$;
1,05 ppm (t, 3H) $CH_3—CH_2$.

Beispiel 18

134 mg (0,5 mMol) Ammoniumperrhenat, 9,25 g (50 mMol) 2-Azido-pentansäureisopropylester, 3,85 g (52,6 mMol) Dimethylformamid und 50 mg (0,45 mMol) Hydrochinon wurden in 50 ml Acetonitril gelöst. Dann wurden bei Raumtemperatur unter kräftigem Rühren 12,35 g (52,5 mMol) 2,6-Dichlorzimtsäurechlorid

zugegeben. Das Reaktionsgemisch wurde 16 Stunden bei Raumtemperatur gerührt, wobei das Produkt ausfiel. Der Rückstand wurde abgetrennt, mit Wasser Chlorid-frei gewaschen und getrocknet.

Es ergaben sich 12,5 g (70,2% der Theorie) analysenreiner 2-[2'-(2,6-Dichlorphenyl)-vinylcarbonylamino]-2-pentensäureisopropylester.

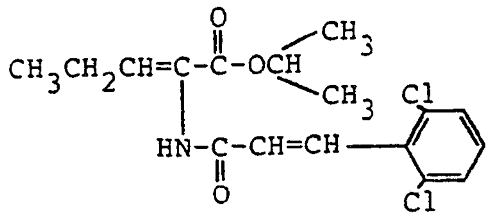

$C_{17}H_{19}NO_3Cl_2$(356,27)

| | %C | %H | %N | %Cl |
|---|---|---|---|---|
| Berechnet: | 57,31 | 5,37 | 3,53 | 19,90 |
| Gefunden: | 57,31 | 5,76 | 4,06 | 18,45 |

Schmelzpunkt: 166°C

$^1$H—NMR ($CDCl_3$/TMS): δ =

7,75 (d, 1H) —COCH=;
6,85—7,5 (m, 4H) NH; aromat. CH;
6,65 (d, 1H) —C=CH—;
6,55 (t, 1H) $CH_2$—*CH*—;
4,65—5,3 (m, 1H) COOCH—;
1,85—2,5 (m, 2H) —$CH_2$—;
1,25 (d, 6H) C—$(CH_3)_2$;
1,05 ppm (t, 3H) $CH_3$—.

## Beispiel 19

27,3 mg (0,1 mMol) Natriumperrhenat, 1,71 g (10 mMol) 2-Azido-hexansäuremethylester und 767,6 mg (10,5 mMol) Dimethylformamid wurden in 1 ml Acetonitril gelöst. Dann wurden bei Raumtemperatur unter kräftigem Rühren 1,19 g (10,5 mMol) Chloracetylchlorid zugegeben. Der Ansatz reagierte innerhalb 30 Minuten exotherm ab, er wurde anschließend analog Beispiel 1 aufgearbeitet.

Es ergaben sich 1,8109 g (82,4% der Theorie) analysenreiner 2-Chlormethylcarboxamido-2-hexensäuremethylester.

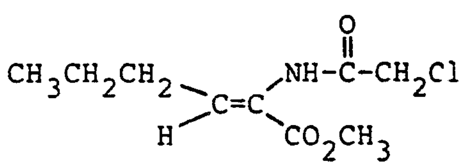

$C_9H_{14}ClNO_3$(219,668)

| | %C | %H | %N | %Cl |
|---|---|---|---|---|
| Berechnet: | 49,21 | 6,42 | 6,38 | 16,14 |
| Gefunden: | 49,21 | 6,33 | 6,41 | 16,15 |

Schmelzpunkt: 63,5 bis 64°C

$^1$H—NMR ($CDCl_3$): δ =

8,0 (s̄, 1H) NH;
6,86 (t, 1H)—$CH_2$—CH=;
4,19 (s, 2H) $CH_2Cl$;
3,81 (s, 3H) $OCH_3$;
2,18 (q, 2H)—$CH_2$—CH=;
1,8—1,28 (m, 2H) $CH_3$—$CH_2$—;
0,95 ppm (t, 3H) $CH_3$—$CH_2$.

IR(KBr) $\nu_{NH}$ = 3255 $cm^{-1}$,
$\nu_{CO}$ = 1722 $cm^{-1}$, 1664 $cm^{-1}$.

## Beispiel 20

134 mg (0,5 mMol) Ammoniumperrhenat, 9,25 g (50 mMol) 2-Azido-hexansäureethylester, 3,85 g (52,6 mMol) Dimethylformamid und 50 mg (0,45 mMol) Hydrochinon wurden in 50 ml Essigsäure-isopropylester gelöst. Dann wurden bei Raumtemperatur unter kräftigem Rühren 5,95 g (52,6 mMol) Chloracetylchlorid zugegeben. Der Ansatz reagierte innerhalb 40 Minuten exotherm ab.

Die Aufarbeitung erfolgte analog Beispiel 16. Es ergaben sich 8,7 g (74,4% der Theorie) analysenreiner 2-Chlormethylcarboxamido-2-hexensäureethylester.

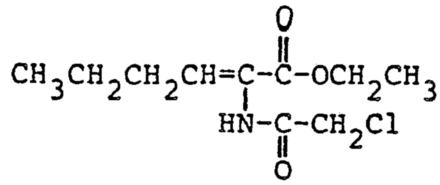

$C_{10}H_{16}NO_3Cl$(233,69)

| | %C | %H | %N | %Cl |
|---|---|---|---|---|
| Berechnet: | 51,40 | 6,90 | 6,0 | 15,17 |
| Gefunden: | 51,56 | 6,83 | 6,06 | 15,23 |

Schmelzpunkt: 49 bis 49,5°C
$^1$H—NMR ($CDCl_3$/TMS): δ =
7,9 (s breit, 1H) NH;
6,75 (t, 1H) —CH=;
4,2 (q, 2H) —$CH_2Cl$;
1,8—2,4 (m, 2H) —$CH_2$—CH=;
1,2—1,8 (m, 2H) $CH_3$—$CH_2$—;
1,3 (t, 3H) $COOCH_2CH_3$;
0,9 ppm (t, 3H) $CH_3$—$CH_2$.

## Beispiel 21

a) 134 mg (0,5 mMol) Ammoniumperrhenat, 9,25 g (50 mMol) 2-Azido-hexansäureethylester, 5,2 g (52,5 mMol) N-Methylpyrrolidon und 50 mg (0,45 mMol) Hydrochinon wurden in 50 ml Acetonitril vorgelegt. Dann wurden bei Raumtemperatur unter kräftigem Rühren 7,75 g (52,5 mMol) Dichloracetylchlorid zugegeben. Der Ansatz reagierte innerhalb 20 Minuten exotherm ab. Die Aufarbeitung erfolgte analog Beispiel 3.

Es ergaben sich 10,1 g (75,3%) analysenreiner 2-Dichlormethylcarboxamido-2-hexensäureethylester.

b) 134 mg (0,5 mMol) Ammoniumperrhenat, 9,25 g (50 mMol) 2-Azido-hexansäureethylester, 6,1 g (52,5 mMol) Tetramethylharnstoff und 50 mg Hydrochinon wurden analog a) mit 7,75 g (52,5 mMol) Dichloracetylchlorid umgesetzt. Es ergaben sich 9,4 g (70,1% der Theorie) analysenreiner 2-Dichlormethylcarboxamido-2-hexensäureethylester.

c) 134 mg (0,5 mMol) Ammoniumperrhenat, 9,25 g (50 mMol) 2-Azido-hexansäureethylester, 9,4 g (52,5 mMol) N,N,N',N', N'',N''-Hexamethylphosphorsäuretriamid und 50 mg (0,45 mMol) Hydrochinon wurden analog a) mit 7,75 g (52,5 mMol) Dichloracetylchlorid umgesetzt. Es ergaben sich 7,3 g (54,4% der Theorie) analysenreiner 2-Dichlormethylcarboxamido-2-hexensäureethylester.

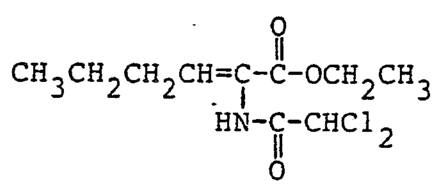

$C_{10}H_{15}NO_3Cl_2$(268,14)

| | %C | %H | %N | %Cl |
|---|---|---|---|---|
| Berechnet: | 44,79 | 5,64 | 5,22 | 26,44 |
| Gefunden: | 44,61 | 5,30 | 5,20 | 26,31 |

Schmelzpunkt: 75°C
$^1$H—NMR ($CDCl_3$/TMS): δ =
7,9 (s breit, 1H) NH;
6,8 (t, 1H) —CH=;
6,05 (s, 1H) —$CHCl_2$;
4,25 (q, 2H) $COOCH_2$—;
1,9—2,4 (m, 2H) —$CH_2$—CH=;
1,2—1,85 (m, 2H) $CH_3$—$CH_2$—;
1,35 (t, 3H) —$COOCH_2CH_3$;
0,95 ppm (t, 3H) $CH_3$—$CH_2$.

Beispiel 22

134 mg (0,5 mMol) Ammoniumperrhenat, 9,95 g (49,9 mMol) 2-Azido-octansäuremethylester, 3,85 g (52,5 mMol) Dimethylformamid und 50 mg (0,45 mMol) Hydrochinon wurden in 50 ml Acetonitril gelöst. Dann wurden bei Raumtemperatur unter kräftigem Rühren 5,95 g (52,6 mMol) Chloracetylchlorid zugegeben. Der Ansatz reagierte innerhalb 25 Minuten exotherm ab.

Die Aufarbeitung erfolgte analog Biespiel 3. Es ergaben sich 10,8 g (87,3% der Theorie) analysenreiner 2-Chlormethylcarboxamido-2-octensäuremethylester.

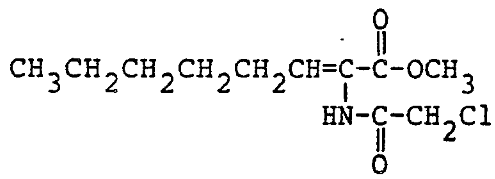

$C_{11}H_{18}NO_3Cl$(247,72)

| | %C | %H | %N | %Cl |
|---|---|---|---|---|
| Berechnet: | 53,33 | 7,32 | 5,65 | 14,31 |
| Gefunden: | 53,29 | 7,41 | 5,52 | 14,27 |

Schmelzpunkt: 52,5°C

$^1H$—NMR ($CDCl_3$/TMS): $\delta$ =

7,85 (s breit, 1H) NH;
6,8 (t, 1H) —CH=;
4,2 (s, 2H) —$CH_2Cl$;
3,85 (s, 3H) —$COOCH_3$;
2,2 (dt, 2H) —$CH_2CH$=;
0,65—1,85 ppm (m, 9H) $CH_3CH_2CH_2CH_2$—.

Beispiel 23

246 mg (0,5 mMol) Tetra-n-butyl-ammoniumperrhenat, 9,95 g (49,9 mMol) 2-Azidooctansäuremethylester, 3,85 g (52,6 mMol) Dimethylformamid und 50 mg (0,45 mMol) Hydrochinon wurden in 50 ml Acetonitril gelöst. Dann wurden bei Raumtemperatur unter kräftigem Rühren 7,75 g (52,5 mMol) Dichloracetylchlorid zugegeben. Der Ansatz reagierte sofort exotherm ab.

Die Aufarbeitung erfolgte analog Beispiel 3. Es ergaben sich 10,4 g (73,85% der Theorie) analysenreiner 2-Dichlormethylcarboxamido-2-octensäuremethylester.

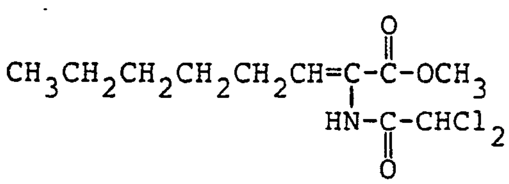

$C_{11}H_{17}NO_3Cl_2$(282,17)

| | %C | %H | %N | %Cl |
|---|---|---|---|---|
| Berechnet: | 46,82 | 6,07 | 4,96 | 25,13 |
| Gefunden: | 46,88 | 6,22 | 4,87 | 24,63 |

Schmelzpunkt: 57°C

$^1H$—NMR ($CDCl_3$/TMS): $\delta$ =

7,8 (s breit, 1H) NH;
6,75 (t, 1H) —CH=;
5,95 (s, 1H) —$CHCl_2$;
3,8 (s, 3H) —$COOCH_3$;
2,15 (dt, 2H) —$CH_2$—CH;
0,55—1,75 ppm (m, 9H) $CH_3CH_2CH_2CH_2$—.

Beispiel 24

246 mg (0,5 mMol) Tetra-n-butyl-ammoniumperrhenat, 11,35 g (49,9 mMol) 2-Azido-octansäureisopropylester, 3,85 g (52,6 mMol) Dimethylformamid und 50 mg (0,45 mMol) Hydrochinon wurden in 50 ml Essigsäureethylester gelöst. Dann wurden bei Raumtemperatur unter kräftigem Rühren 5,95 g (52,6 mMol) Chloracetylchlorid zugegeben. Der Ansatz reagierte innerhalb 1 Stunde exotherm ab.

Die Aufarbeitung erfolgte analog Beispiel 16. Es ergaben sich 10,5 g (76,3% der Theorie) analysenreiner 2-Chlormethylcarboxamido-2-octensäureisopropylester.

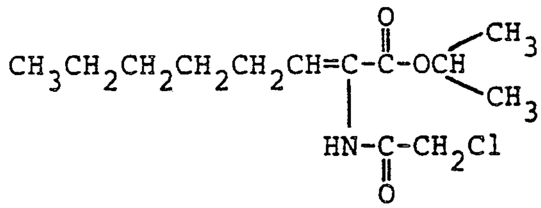

$C_{13}H_{22}NO_2Cl$(275,78)

| | %C | %H | %N | %Cl |
|---|---|---|---|---|
| Berechnet: | 56,62 | 8,04 | 5,079 | 12,85 |
| Gefunden: | 56,55 | 8,32 | 5,00 | 12,83 |

Schmelzpunkt: 55°C
$^1$H—NMR ($CDCl_3$/TMS): δ =
7,85 (s breit, 1H) NH;
6,7 (t, 1H) —CH=;
5,35—4,7 (m, 1H) —COOCH—;
4,1 (s, 2H) —$CH_2Cl$;
2,15 (dt, 2H) —$CH_2$—CH=;
1,35 (d, 6H) —CH—$(CH_3)_2$;
0,5—1,8 ppm (m, 9H) $CH_3CH_2CH_2CH_2$—.

Beispiel 25

246 mg (0,5 mMol) Tetra-n-butyl-ammoniumperrhenat, 11,35 g (49,9 mMol) 2-Azido-octansäureisopropylester, 3,85 g (52,6 mMol) Dimethylformamid und 50 mg (0,45 mMol) Hydrochinon wurden in 50 ml Acetonitril gelöst. Dann wurden bei Raumptemperatur unter kräftigem Rühren 7,75 g (52,5 mMol) Dichloracetylchlorid zugegeben. Das Reaktionsgemisch wurde 18 Stunden bei Raumtemperatur gerührt und anschließend analog Beispiel 3 aufgearbeitet.

Es ergaben sich 12,3 (79,3% der Theorie) analysenreiner 2-Dichlormethylcarboxamido-2-octensäureisopropylester.

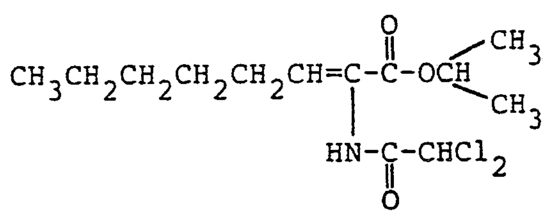

$C_{13}H_{21}NO_3Cl_2$(310,22)

| | %C | %H | %N | %Cl |
|---|---|---|---|---|
| Berechnet: | 50,33 | 6,82 | 4,51 | 22,85 |
| Gefunden: | 50,11 | 6,96 | 4,50 | 22,77 |

Schmelzpunkt: 88°C
$^1$H—NMR ($CDCl_3$/TMS): δ =
7,95 (s breit, 1H) NH;
6,8 (t, 1H) —CH=;
6,05 (s, 1H) —$CHCl_2$;
5,4—4,7 (m, 1H) —COOCH—;
2,15 (dt, 2H) —$CH_2$—CH=;
1,35 (d, 6H) —CH—$(CH_3)_2$;
0,6—1,8 ppm (m, 9H) $CH_3CH_2CH_2CH_2$—.

Beispiel 26

134 mg (0,5 mMol) Ammoniumperrhenat, 9,85 g (49,9 mMol) 2-Azido-3-cyclopentylpropionsäuremethylester, 3,85 g (52,6 mMol) Dimethylformamid und 50 mg (0,45 mMol) Hydrochinon wurden in 50 ml Acetonitril gelöst. Dann wurden bei Raumtemperatur unter kräftigem Rühren 5,93 g (52,5 mMol) Chloracetylchlorid zugegeben. Das Reaktionsgemisch wurde 13 Stunden bei Raumtemperatur gerührt und anschließend analog Beispiel 3 aufgearbeitet.

Es ergaben sich 6,9 g (56,3% der Theorie) analysenreiner 2-Chlormethylcarboxamido-3-cyclopentyl-propensäuremethylester.

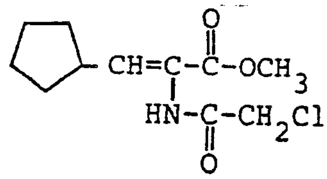

$C_{11}H_{16}NO_3Cl$(245, 71)

| | %C | %H | %N | %Cl |
|---|---|---|---|---|
| Berechnet: | 53,77 | 6,56 | 5,70 | 14,43 |
| Gefunden: | 53,61 | 6,63 | 5,29 | 14,78 |

Schmelzpunkt: 65,5°C
$^1$H—NMR ($CDCl_3$/TMS): δ =
7,8 (s breit, 1H) NH;
6,7 (d, 1H) —CH=;
4,15 (s, 2H) —$CH_2Cl$;
3,8 (s, 3H) $COOCH_3$;
2,15—3,1 (s breit, 1H) 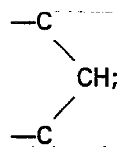
1,0—2,2 ppm (m, 8H) 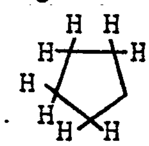

Beispiel 27

134 mg (0,5 mMol) Ammoniumperrhenat, 9,85 g (49,9 mMol) 2-Azido-3-cyclopentyl-propionsäuremethylester, 3,85 g (52,6 mMol) Dimethylformamid und 50 mg (0,45 mMol) Hydrochinon wurden in 50 ml Acetonitril gelöst. Dann wurden bei Raumtemperatur unter kräftigem Rühren 7,75 g (52,5 mMol) Dichloracetylchlorid zugegeben. Das Reaktionsgemisch wurde 15 Stunden bei Raumtemperatur gerührt und anschließend analog Beispiel 3 aufgearbeitet.

Es ergaben sich 10,9 g (77,9% der Theorie) analysenreiner 2-Dichlormethylcarboxamido-3-cyclopentyl-propensäuremethylester.

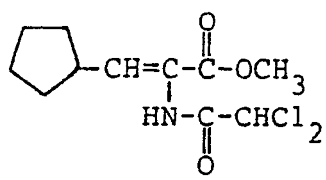

$C_{11}H_{15}NO_3Cl_2$(280,15)

| | %C | %H | %N | %Cl |
|---|---|---|---|---|
| Berechnet: | 47,16 | 5,40 | 5,00 | 25,31 |
| Gefunden: | 47,06 | 5,32 | 4,98 | 25,00 |

Schmelzpunkt: 85 bis 86°C
$^1$H—NMR ($CDCl_3$/TMS): δ =
7,75 (s breit, 1H) NH;
6,75 (d, 1H) —CH=;
6,0 (s, 1H) —$CHCl_2$;
3,8 (s, 3H) $COOCH_3$;
2,25—3,0 (s breit, 1H) 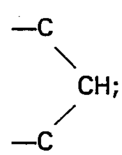
0,9—2,2 ppm (m, 8H) 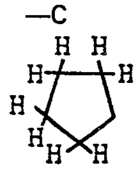

Beispiel 28

134 mg (0,5 mMol) Ammoniumperrhenat, 11,25 g (49,9 mMol) 2-Azido-3-cyclopentyl-propionsäureisopropylester, 3,85 g (52,6 mMol) Dimethylformamid und 50 mg (0,45 mMol) Hydrochinon wurden in 50 ml Acetonitril gelöst. Dann wurden bei Raumtemperatur unter kräftigem Rühren 5,93 g (52,5 mMol) Choracetylchlorid zugegeben. Der Ansatz reagierte innerhalb 30 Minuten exotherm ab.

Die Aufarbeitung erfolgte analog Beispiel 3. Es ergaben sich 11,2 g (81,8% der Theorie) analysenreiner 2-Chlormethylcarboxamido-3-cyclopentyl-propensäureisopropylester.

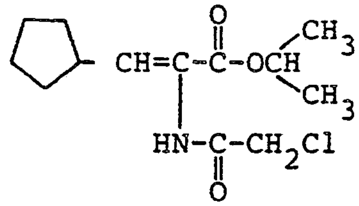

$C_{13}H_{20}NO_3Cl$(273,76)

| | %C | %H | %N | %Cl |
|---|---|---|---|---|
| Berechnet: | 57,04 | 7,36 | 5,12 | 12,95 |
| Gefunden: | 57,04 | 7,71 | 5,15 | 13,01 |

Schmelzpunkt: 72°C

$^1$H—NMR ($CDCl_3$/TMS): δ =

7,75 (s breit, 1H) NH;

6,65 (d, 1H) —CH=;

4,7—5,35 (m, 1H) COOCH;

4,15 (s, 2H) —$CH_2Cl$;

2,25—3,0 (s breit, 1H) 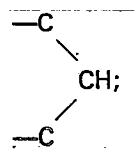

— 2,15 (m, 8H) 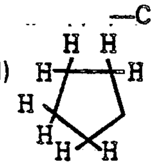

1,30 ppm (d, 6H) 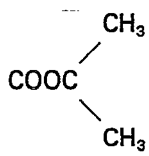

Beispiel 29

134 mg (0,5 mMol) Ammoniumperrhenat, 11,25 g (49,9 mMol) 2-Azido-3-cyclopentyl-propionsäureisopropylester, 3,85 g (52,6 mMol) Dimethylformamid und 50 mg (0,45 mMol) Hydrochinon wurden in 50 ml Acetonitril gelöst. Dann wurden bei Raumtemperatur unter kräftigem Rühren 7,75 g (52,5 mMol) Dichloracetylchlorid zugegeben. Der Ansatz reagierte innerhalb 45 Minuten exotherm ab.

Die Aufarbeitung erfolgte analog Beispiel 3. Es ergaben sich 8,7 g (56,6% der Theorie) analysenreiner 2-Dichlormethylcarboxamido-3-cyclopentyl-propensäureisopropylester.

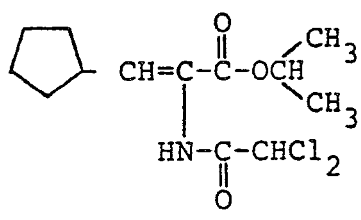

$C_{13}H_{19}NO_3Cl_2$(308, 205)

| | %C | %H | %N | %Cl |
|---|---|---|---|---|
| Berechnet: | 50,66 | 6,21 | 4,54 | 23,00 |
| Gefunden: | 50,82 | 6,26 | 4,63 | 22,79 |

Schmelzpunkt: 99°C

$^1H$—NMR ($CDCl_3$/TMS): δ =

7,85 (s breit, 1H) NH;

6,75 (d, 1H) —CH=;

6,05 (s, 1H) $CHCl_2$;

4,7—5,4 (m, 1H) COOCH;

2,25—2,95 (s breit, 1H) 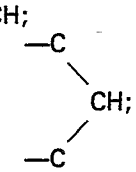

– 2,2 (m, 8H) 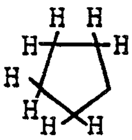

1,35 ppm (d, 6H) 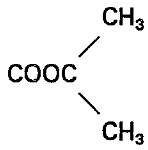

## Beispiel 30

134 mg (0,5 mMol) Ammoniumperrhenat, 10,55 g (50 mMol) 2-Azido-3-cyclohexylpropionsäuremethylester, 3,85 g (52,6 mMol) Dimethylformamid und 50 mg (0,45 mMol) Hydrochinon wurden in 50 ml Essigsäure-n-propylester gelöst. Dann wurden bei Raumptemperatur unter kräftigem Rühren 5,93 g (52,5 mMol) Chloracetylchlorid zugegeben. Das Reaktionsgemisch erreichte nach 1 Stunde eine maximale Reaktionsexotherme von 45°C. Die Aufarbeitung erfolgte analog Beispiel 16. Es ergaben sich 10,3 g (79,3% der Theorie) analysenreiner 2-Chlormethylcarboxamido-3-cyclohexylpropensäuremethylester.

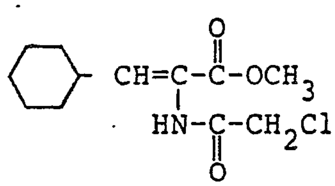

$C_{12}H_{18}NO_3Cl$(259,73)

| | %C | %H | %N | %Cl |
|---|---|---|---|---|
| Berechnet: | 55,49 | 6,99 | 5,39 | 13,65 |
| Gefunden: | 55,39 | 7,28 | 4,75 | 13,83 |

Schmelzpunkt: 117 bis 118°C

$^1H$—NMR ($CDCl_3$/TMS): δ =

7,7 (s breit, 1H) NH;

6,6 (d, 1H) —CH=;

4,15 (s, 2H) —$CH_2Cl$;

3,85 (s, 3H) $COOCH_3$;

0,75—2,6 ppm (m, 11H) 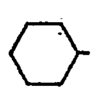.

## Beispiel 31

134 mg (0,5 mMol) Ammoniumperrhenat, 10,55 g (50 mMol) 2-Azido-3-cyclohexylpropionsäuremethylester, 3,85 g (52,6 mMol) Dimethylformamid und 50 mg (0,45 mMol) Hydrochinon wurden in 50 ml Acetonitril gelöst. Dann wurden bei Raumtemperatur 7,75 g (52,5 mMol) Dichloracetylchlorid unter kräftigem Rühren zugegeben. Das Reaktionsgemisch wurde 16 Stunden bei Raumtemperatur gerührt und anschließend analog Beispiel 3 aufgearbeitet.

Es ergaben sich 11,7 g (79,5% der Theorie) analysenreiner 2-Dichlormethylcarboxamido-3-cyclohexyl-propensäuremethylester.

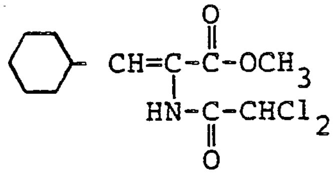

$C_{12}H_{17}NO_3Cl_2$(294,18)

| | %C | %H | %N | %Cl |
|---|---|---|---|---|
| Berechnet: | 48,99 | 5,82 | 4,76 | 24,10 |
| Gefunden: | 48,95 | 5,91 | 4,70 | 24,06 |

Schmelzpunkt: 98°C
$^1H$—NMR ($CDCl_3$/TMS): δ =
7,65 (s breit, 1H) NH;
6,65 (d, 1H) —CH=;
5,95 (s, 1H) —$CH_2Cl$);
3,8 (s, 3H) $COOCH_3$;

1,9—2,65 (s breit, 1H) 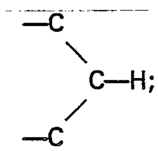

0,7—2,0 ppm (m, 10H) 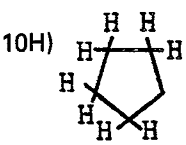

Beispiel 32

13,7 mg (0,05 mMol) Natriumperrhenat, 715,7 mg (5 mMol) 2-Azido-butansäuremethylester und 383,8 mg (5,25 mMol) Dimethylformamid wurden in 5 ml Acetonitril gelöst. Dann wurden bei Raumtemperatur unter kräftigem Rühren 1,195 g (5,25 mMol) 2-(2′,2′-Dichlorethenyl)-3,3-dimethyl-cyclopropancarbonsäurechlorid zugegeben. Das Reaktionsgemisch wurde 30 Stunden bei Raumtemperatur gerührt und anschließend analog Beispiel 1 aufgearbeitet.

Es ergaben sich 1,31 g (85,6% der Theorie) analysenreiner 2-[2′-(2,2-Dichlorethenyl)-3′,3′-dimethyl]-cyclopropancarboxamido-2-hexensäuremethylester.

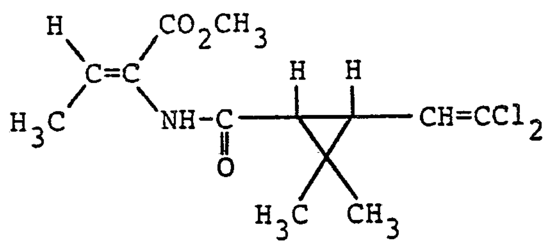

$C_{13}H_{17}Cl_2NO_3$(306,189)

| | %C | %H | %N | %Cl |
|---|---|---|---|---|
| Berechnet: | 51,00 | 5,60 | 4,57 | 23,16 |
| Gefunden: | 50,79 | 5,66 | 4,29 | 23,11 |

Schmelzpunkt: 109 bis 109,5°C
$^1H$-NMR ($CDCl_3$): δ =
7,32 (s, 1H) NH;
6,8 (q, 1H) H—C($CH_3$)=;
6,42 (d, 1H) H—C=$CCl_2$;
3,77 (s, 3H) $OCH_3$;
1,76 (d, 3H) $H_3C$—CH=;

1,28 (s, 3H)
1,25 ppm (s, 3H) } 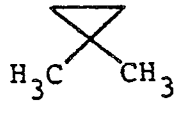

IR $\nu_{NH}$ = 3320 $cm^{-1}$,
$\nu_{CO}$ = 1720 $cm^{-1}$, 1658 $cm^{-1}$.

Beispiel 33

13,7 mg (0,05 mMol) Natriumperrhenat, 856 mg (5 mMol) 2-Azido-hexansäuremethylester und 838,8 mg (5,25 mMol) Dimethylformamid wurden in 0,5 ml Acetonitril gelöst. Dann wurden bei Raumtemperatur unter kräftigem Rühren 1,1945 g (5,25 mMol) 2-(2',2'-Dichlorethenyl)-3,3-dimethyl-cyclopropancarbonsäurechlorid zugegeben. Das Reaktionsgemisch wurde 15 Stunden bei Raumtemperatur gerührt und anschließend analog Beispiel 1 aufgearbeitet.

Es ergaben sich 1,40 g (83,8% der Theorie) analysenreiner 2-[2'-(2,2-Dichlorethenyl)-3',3'-dimethyl]-cyclopropancarboxamido-2-hexensäuremethylester.

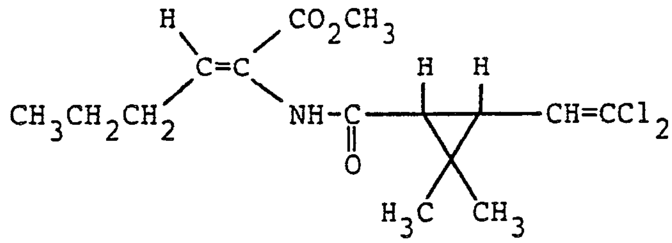

$C_{15}H_{21}Cl_2NO_3$(334,243)

| | %C | %H | %N | %Cl |
|---|---|---|---|---|
| Berechnet: | 53,90 | 6,33 | 4,19 | 21,21 |
| Gefunden: | 53,62 | 6,51 | 3,98 | 21,44 |

Schmelzpunkt: 105 bis 106°C

$^1H$—NMR ($CDCl_3$): δ =
6,97 (s, 1H) NH;
6,73 (t, 1H) —$CH_2$—CH=;
6,43 (d, 1H) H—C=$CCl_2$;
3,8 (s, 3H) $OCH_3$;
2,38—0,77 (m, 9H) $CH_3CH_2CH_2$; 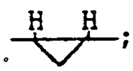;
1,3 (s, 3H)
1,27 ppm (s, 3H) } 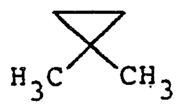

IR $\nu_{NH}$ = 3300 $cm^{-1}$,
$\nu_{CO}$ = 1715 $cm^{-1}$, 1655 $cm^{-1}$.

Beispiel 34

13,7 mg (0,05 mMol) Natriumperrhenat, 1,0261 g (5 mMol) 2-Azido-3-phenyl-propansäuremethylester und 383,8 mg (5,25 mMol) Dimethylformamid wurden in 5 ml Acetonitril gelöst. Dann wurden bei Raumtemperatur unter kräftigem Rühren 592,9 mg (5,25 mMol) Chloracetylchlorid zugegeben. Das Reaktionsgemische wurde 7 Stunden bei Raumtemperatur gerührt und anschließend analog Beispiel 1 aufgearbeitet.

Es ergaben sich 1,1657 g (91,9% der Theorie) analysenreiner 2-Chlormethylcarboxamido-3-phenylpropensäuremethylester.

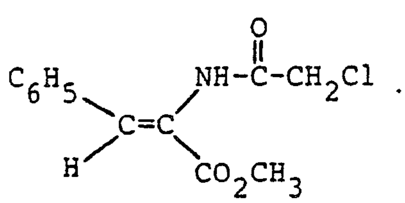

$C_{12}H_{12}ClNO_3$(253,685)

| | %C | %H | %N | %Cl |
|---|---|---|---|---|
| Berechnet: | 56,82 | 4,77 | 5,52 | 13,98 |
| Gefunden: | 56,90 | 4,83 | 5,38 | 13,93 |

Schmelzpunkt: 113,5 bis 114°C

$^1H$—NMR ($CDCl_3$): δ =
8,12 (s, 1H) NH;
7,7—7,17 (m, 5H) aromat. —CH=;
4,09 (s, 2H) $CH_2Cl$;
3,82 ppm (s, 3H) $CH_3$.
IR $\nu_{NH}$ = 3235 $cm^{-1}$,
$\nu_{CO}$ = 1720 $cm^{-1}$, 1617 $cm^{-1}$.

## Beispiel 35

13,7 g (0,05 mMol) Natriumperrhenat, 1,0261 g (5 mMol) 2-Azido-3-phenyl-propansäuremethylester und 383,8 mg (5,25 mMol) Dimethylformamid wurden in 5 ml Acetonitril gelöst. Dann wurden bei Raumtemperatur unter kräftigem Rühren 1,1945 g (5,25 mMol) 2-(2',2'-Dichlorethenyl)-3,3-dimethylcyclopropancarbonsäurechlorid zugegeben. Das Reaktionsgemisch wurde 15 Stunden bei Raumtemperatur gerührt und anschließend analog Beispiel 1 aufgearbeitet.

Es ergaben sich 1,7136 g (93,1% der Theorie) analysenreiner 2 - [2' - (2,2 - Dichlorethenyl) - 3',3' - dimethyl] - cyclopropancarboxamido - 3 - phenyl - propensäuremethylester.

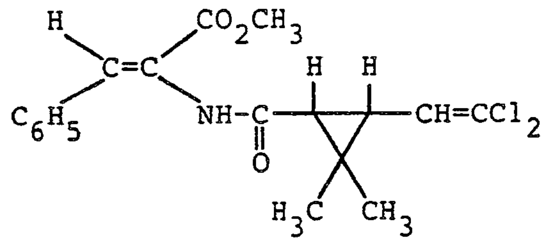

$C_{18}H_{19}Cl_2NO_3$(368,26)

| | %C | %H | %N | %Cl |
|---|---|---|---|---|
| Berechnet: | 58,71 | 5,20 | 3,80 | 19,25 |
| Gefunden: | 58,61 | 5,12 | 3,54 | 19,27 |

Schmelzpunkt: 127 bis 128°C.

$^1H$—NMR ($CDCl_3$): δ =

7,65—7,15 (m, 5H) aromat.—CH=;

6,35 (d, 1H) —$CH=CCl_2$;

3,85 (s, 3H) $OCH_3$;

1,25 (s, 3H)

1,23 ppm (s, 3H)

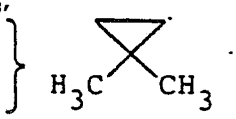

IR $\nu_{NH}$ = 3270 $cm^{-1}$,

$\nu_{CO}$ = 1720 $cm^{-1}$, 1635 $cm^{-1}$.

## Beispiel 36

27,3 mg (0,1 mMol) Natriumperrhenat und 1,57 g (10 mMol) 2-Azido-3-methyl-butansäuremethylester wurden bei 80°C in 5 ml Essigsäureethylester vorgelegt. Dann wurden innerhalb von 20 Minuten unter kräftigem Rühren 1,19 g (10,5 mMol) Chloracetylchlorid, gelöst in 5 ml Essigsäureethylester, zugetropft. Das Reaktionsgemisch wurde unter kräftigem Rühren insgesamt 3 Stunden auf 80°C gehalten und anschließend analog Beispiel 1 aufgearbeitet.

Es ergaben sich 1,97 g (95,8% der Theorie) analysenreiner 2-Chlormethylcarboxamido-3-methyl-2-butensäuremethylester.

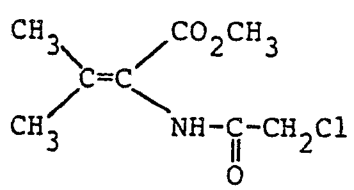

$C_8H_{12}ClNO_3$(205,641)

| | %C | %H | %N | %Cl |
|---|---|---|---|---|
| Berechnet: | 46,73 | 5,88 | 6,81 | 17,24 |
| Gefunden: | 46,76 | 5,79 | 6,75 | 17,34 |

Schmelzpunkt: 106°C
$^1H$—NMR ($CDCl_3$): δ =
7,83 (s, 1H) NH;
4,17 (s, 2H) $CH_2Cl$;
3,8 (s, 3H) $OCH_3$;
2,22 (d, 3H) 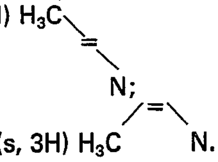
1,89 ppm (s, 3H)
IR $\nu_{NH}$ = 3275 $cm^{-1}$,
$\nu_{CO}$ = 1719 $cm^{-1}$, 1660 $cm^{-1}$.

Beispiel 37

134 mg (0,5 mMol) Ammoniumperrhenat, 8,55 g (49,9 mMol) 2-Azido-3-methyl-butansäureethylester, 4,6 g (52,8 mMol) Dimethylacetamid und 50 mg (0,45 mMol) Hydrochinon wurden in 50 ml Acetonitril vorgelegt. Dann wurden bei Raumtemperatur unter kräftigem Rühren 5,95 g (52,6 mMol) Chloracetylchlorid zugegeben. Der Ansatz reagierte innerhalb 15 Minuten exotherm ab.

Die Aufarbeitung erfolgte analog Beispiel 3. Es ergaben sich 9,35 g (85,3% der Theorie) analysenreiner 2-Chlormethylcarboxamido-3-methyl-2-butensäureethylester.

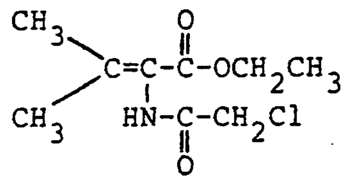

$C_9H_{14}NO_3Cl$(219,67)

| | %C | %H | %N | %Cl |
|---|---|---|---|---|
| Berechnet: | 49,21 | 6,42 | 6,38 | 16,14 |
| Gefunden: | 49,11 | 6,52 | 6,31 | 16,29 |

Schmelzpunkt: 117°C
$^1H$—NMR($CDCl_3$/TMS): δ =
7,7 (s breit, 1H) NH;
4,15 (q, 2H) $COOCH_2$—;
4,1 (s, 2H) —$CH_2Cl$;
2,2 (s, 3H) $CH_3$—C=;
1,85 (s, 3H) $CH_3$—C=;
1,25 ppm (t, 3H) $COOCH_2CH_3$.

Beispiel 38

134 mg (0,5 mMol) Ammoniumperrhenat, 8,55 g (49,9 mMol) 2-Azido-3-methyl-butansäureethylester, 3,85 g (52,6 mMol) Dimethylformamid und 50 mg (0,45 mMol) Hydrochinon wurden in 50 ml Acetonitril vorgelegt. Dann wurden bei Raumtemperatur 7,75 g (52,5 Mol) Dichloracetylchlorid zugegeben. Der Ansatz reagierte innerhalb 40 Minuten exotherm ab.

Die Aufarbeitung erfolgte analog Beispiel 3. Es ergaben sich 9,8 g (77,3% der Theorie) analysenreiner 2-Dichlormethylcarboxamido-3-methyl-2-butensäureethylester.

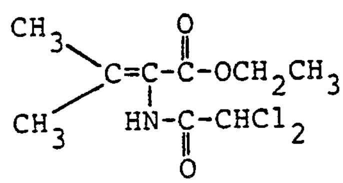

$C_9H_{13}NO_3Cl_2$(254,11)

| | %C | %H | %N | %Cl |
|---|---|---|---|---|
| Berechnet: | 42,54 | 5,16 | 5,51 | 27,90 |
| Gefunden: | 42,20 | 5,19 | 5,44 | 27,58 |

Schmelzpunkt: 145°C
$^1H$—NMR ($CDCl_3$/TMS): δ =
7,8 (s breit, 1H) NH);
5,95 (s, 1H) $CHCl_2$;
4,2 (q, 2H) $COOCH_2$;
2,25 (s, 3H) $CH_3$—C=;
1,9 (s, 3H) $CH_3$—C=;
1,25 ppm (t, 3H) $COOCH_2CH_3$.

Beispiel 39

134 mg (0,5 mMol) Ammoniumperrhenat, 8,55 g (49,9 mMol) 2-Azido-3-methyl-butansäureethylester, 3,85 g (52,6 mMol) Dimethylformamid und 50 mg (0,45 mMol) Hydrochinon wurden in 50 ml Acetonitril vorgelegt. Dann wurden bei Raumtemperatur unter kräftigem Rühren 6,45 g (52,4 mMol) Acetylbromid zugegeben. Das Reaktionsgemisch wurde 3 Stunden gerührt, wobei eine maximale Reaktionstemperatur von 35°C erreicht wurde.

Die Aufarbeitung erfolgte analog Beispiel 3. Es ergaben sich 4,8 g (51,9% der Theorie) analysenreiner N-Acetyl-2,3-dehydro-valinethylester.

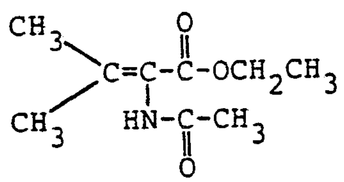

$C_9H_{15}NO_3$(185,22)

| | %C | %H | %N |
|---|---|---|---|
| Berechnet: | 58,36 | 8,16 | 7,56 |
| Gefunden: | 57,71 | 7,88 | 7,52 |

Schmelzpunkt: 77°C
$^1H$—NMR ($CDCl_3$/TMS): δ =
7,9 (s breit, 1H) NH;
4,15 (q, 2H) —$COOCH_2$—;
2,1 (s, 3H) $COCH_3$;
2,05 (s, 3H) $CH_3$—C=;
1,8 (s, 3H) $CH_3$—C=;
1,2 ppm (t, 3H) —$COOCH_2$—$CH_3$.

Beispiel 40

134 mg (0,5 mMol) Ammoniumperrhenat, 8,55 g (49,9 mMol) 2-Azido-3-methyl-butansäureethylester, 3,85 g (52,6 mMol) Dimethylformamid und 50 mg (0,45 mMol) Hydrochinon wurden in 50 ml Acetonitril vorgelegt. Dann wurden bei Raumtemperatur unter kräftigem Rühren 5,7 g (52,5 mMol) Methoxyessigsäurechlorid zugegeben. Das Reaktionsgemisch wurde 70 Stunden bei Raumtemperatur gerührt und anschließend analog Beispiel 3 aufgearbeitet. Das so erhaltene Rohprodukt wurde im Vakuum (0,4 mbar) destilliert.

Es ergaben sich 6,0 g (55,8% der Theorie) analysenreiner N-Methoxyacetyl-2,3-dehydro-valinethylester.

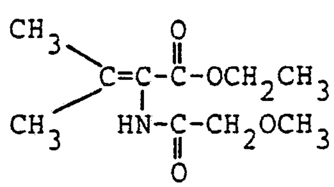

$C_{10}H_{17}NO_4$(215,25)

| | %C | %H | %N |
|---|---|---|---|
| Berechnet: | 55,80 | 7,96 | 6,51 |
| Gefunden: | 55,87 | 8,60 | 6,64 |

Schmelzpunkt: 112 bis 114°C
$^1H$—NMR ($CDCl_3$/TMS): δ =
7,65 (s breit, 1H) NH;
4,22 (q, 2H) —$COOCH_2$—;
3,95 (s, 2H) CO—$CH_2$—O;
3,45 (s, 3H) —$OCH_3$;
2,15 (s, 3H) $CH_3$—C=;
1,85 (s, 3H) $CH_3$—C=;
1,25 ppm (t, 3H) —$COOCH_2$—$CH_3$.

Beispiel 41

134 mg (0,5 mMol) Ammoniumperrhenat, 8,55 g (49,9 mMol) 2-Azido-3-methyl-butansäuremethylester, 3,85 g (52,6 mMol) Dimethylformamid und 50 mg (0,45 mMol) Hydrochinon wurden in 50 ml Acetonitril vorgelegt. Dann wurden bei Raumtemperatur unter kräftigem Rühren 6,35 g (52,6 mMol) 3-Methylbutansäurechlorid zugegeben. Das Reaktionsgemisch wurde 9 Stunden bei Raumtemperatur gerührt und anschließend analog Beispiel 3 aufgearbeitet.

Es ergaben sich 6,5 g (57,3% der Theorie) analysenreiner 2-[(2'-Methylpropyl)-carbonyl-amino]-3-methyl-2-butensäureethylester.

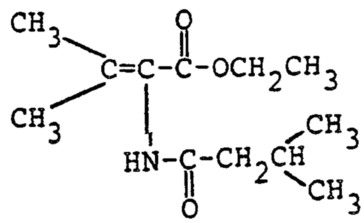

$C_{12}H_{21}NO_3$(227,30)

| | %C | %H | %N |
|---|---|---|---|
| Berechnet: | 63,41 | 9,31 | 6,16 |
| Gefunden: | 64,00 | 10,18 | 6,25 |

Schmelzpunkt: 123 bis 125°C

$^1$H—NMR ($CDCl_3$/TMS): δ =

6,8 (s breit, 1H) NH;
4,2 (q, 2H) —$COOCH_2$—;
1,7—2,4 (m, 3H) $COCH_2$—CH;
2,2 (s, 3H) $CH_3$—C=;
1,9 (s, 3H) $CH_3$—C=;
1,3 (t, 3H) $CH_3$—$CH_2$;
1,05 ppm (d, 6H) 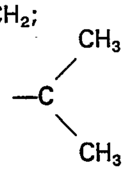 .

Beispiel 42

134 mg (0,5 mMol) Ammoniumperrhenat, 8,55 g (49,9 mMol) 2-Azido-3-methyl-butansäureethylester, 3,85 g (52,6 mMol) Dimethylformamid und 50 mg (0,45 mMol) Hydrochinon wurden in 50 ml Acetonitril vorgelegt. Dann wurden bei Raumtemperatur unter kräftigem Rühren 7,4 g (52,5 mMol) 4-Chlorbutansäurechlorid zugegeben. Das Reaktionsgemisch wurden 10 Stunden bei Raumtemperatur gerührt und anschließend analog Beispiel 3 aufgearbeitet.

Es ergaben sich 8,4 g (67,9% der Theorie) analysenreiner 2-[(3'-Chlorpropyl)-carbonylamino]-3-methyl-2-butensäureethylester.

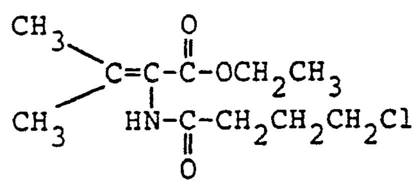

$C_{11}H_{18}NO_3Cl$(247,72)

| | %C | %H | %N | %Cl |
|---|---|---|---|---|
| Berechnet: | 53,33 | 7,32 | 5,65 | 14,31 |
| Gefunden: | 53,36 | 7,64 | 5,67 | 14,50 |

Schmelzpunkt: 80°C

$^1$H—NMR ($CDCl_3$/TMS): δ =

7,1 (s breit, 1H) NH;
4,15 (q, 2H) —$COOCH_2$—;
3,6 (t, 2H) —$CH_2Cl$;
1,75—2,7 (m, 4H) —$COCH_2CH_2$—;
2,15 (s, 3H) $CH_3$—C=;
1,85 (s, 3H) $CH_3$—C=;
1,25 ppm (t, 3H) —$COCCH_2$—$CH_3$.

Beispiel 43

134 mg (0,5 mMol) Ammoniumperrhenat, 8,55 g (49,9 mMol) 2-Azido-3-methyl-butansäureethylester und 3,85 g (52,6 mMol) Dimethylformamid wurden in 50 ml Essigsäureethylester gelöst. Dann wurden bei Raumtemperatur unter kräftigem Rühren 9,2 g (52,5 mMol) 2-Chlor-benzoylchlorid zugegeben. Das Reaktionsgemisch wurde 14 Stunden bei Raumtemperatur gerührt und anschließend analog Beispiel 16 aufgearbeitet.

Es ergaben sich 7,1 g (50,5% der Theorie) analysenreiner 2-[(2'-Chlorphenyl)-carbonyl-amino]-3-methyl-2-butensäureethylester.

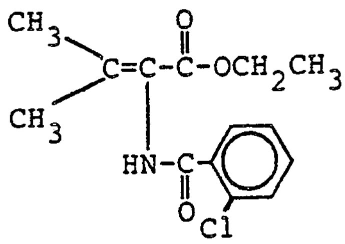

$C_{14}H_{16}NO_3Cl$(281,74)

| | %C | %H | %N | %Cl |
|---|---|---|---|---|
| Berechnet: | 59,68 | 5,72 | 4,97 | 12,58 |
| Gefunden: | 58,72 | 5,96 | 4,91 | 11,64 |

Schmelzpunkt: 86 bis 89°C.

$^1$H—NMR ($CDCl_3$/TMS): δ =
7,0—7,9 (m, 5H) NH; aromat, —CH;
4,2 (q, 2H) $COOCH_2$;
2,2 (s, 3H) $CH_3$—C=;
1,95 (s, 3H) $CH_3$—C=;
1,25 ppm (t, 3H) $COOCH_2CH_2$.

Beispiel 44

134 mg (0,5 mMol) Ammoniumperrhenat, 8,55 g (49,9 mMol) 2-Azido-3-methyl-butansäureethylester, 3,85 g (52,6 mMol) Dimethylformamid und 50 mg (0,45 mMol) Hydrochinon wurden in 50 ml Essigsäureethylester gelöst. Dann wurden bei Raumtemperatur unter kräftigem Rühren 9,2 g (52,5 mMol) 3-Chlorbenzoylchlorid zugegeben. Das Rekationsgemisch wurden 14 Stunden bei Raumtemperatur gerührt und anschließend analog Beispiel 16 aufgearbeitet.

Es ergaben sich 10,2 g (72,5% der Theorie) analysenreiner 2-[(3'-Chlorphenyl)-carbonyl-amino]-3-methyl-2-butensäureethylester.

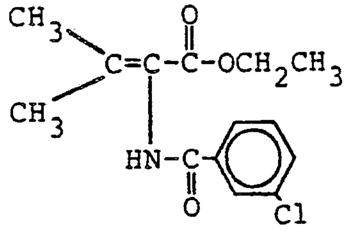

$C_{14}H_{16}NO_3Cl$(281,74)

| | %C | %H | %N | %Cl |
|---|---|---|---|---|
| Berechnet: | 59,68 | 5,72 | 4,97 | 12,58 |
| Gefunden: | 59,52 | 5,78 | 4,89 | 12,32 |

Schmelzpunkt: 120 bis 122°C

$^1$H—NMR ($CDCl_3$/TMS): δ =
7,0—8,1 (m, 5H) NH; aromat.—CH
4.15 (q, 2H) $COOCH_2$;
2,15 (s, 3H) $CH_3$—C=;
1,85 (s, 3H) $CH_3$—C=;
1,25 ppm (t, 3H) $COOCH_2CH_3$.

Beispiel 45

134 mg (0,5 mMol) Ammoniumperrhenat, 8,55 g (49,9 mMol) 2-Azido-3-methyl-butansäureethylester, 3,85 g (52,6 mMol) Dimethylformamid und 50 mg (0,45 mMol) Hydrochinon wurden in 50 ml

Essigsäureethylester gelöst. Dann wurden bei Raumtemperatur unter kräftigem Rühren 9,2 g (52,5 MMol) 4-Chlorbenzoylchlorid zugegeben. Das Reaktionsgemisch wurde 14 Stunden bei Raumtemperatur gerührt. Das ausgefallene Rohprodukt wurde abgesaugt und mit Wasser Chlorid-frei gewaschen.

Es ergaben sich 11,1 g (78,9% der Theorie) analysenreiner 2-[(4'-Chlorphenyl)-carbonyl-amino]-3-methyl-2-butensäureethylester.

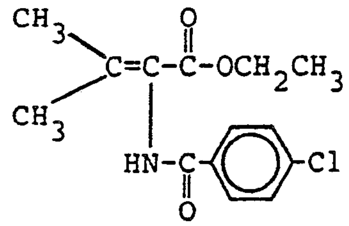

$C_{14}H_{16}NO_3Cl$(281,74)

| | %C | %H | %N | %Cl |
|---|---|---|---|---|
| Berechnet: | 59,68 | 5,72 | 4,97 | 12,58 |
| Gefunden: | 58,97 | 5,55 | 4,09 | 14,08 |

Schmelzpunkt: 124 bis 124,5°C

$^1H$—NMR ($CDCl_3$/TMS): δ =
7,1—8,25 (m, 5H) NH; aromat.—CH;
4,2 (q, 2H) $COOCH_2$;
2,2 (s, 3H) $CH_3$—C=;
1,9 (s, 3H) $CH_3$—C=;
1,25 ppm (t, 3H) $COOCH_2CH_3$.

Beispiel 46

134 mg (0,5 mMol) Ammoniumperrhenat, 8,55 g (49,9 mMol) 2-Azido-3-methyl-butansäureethylester, 3,85 g (52,6 mMol) Dimethyformamid und 50 mg (0,45 mMol) Hydrochinon wurden in 50 ml Essigsäureethylester gelöst. Dann wurden bei Raumtemperatur unter kräftigem Rühren 8,35 g (52,6 mMol) 2-Fluorbenzoylchlorid zugegeben. Das Reaktionsgemisch wurde 14 Stunden bei Raumtemperatur gerührt und anschließend analog Beispiel 16 aufgearbeitet.

Es ergaben sich 9,0 g (68% der Theorie) analysenreiner 2-[(2'-Fluorphenyl)-carbonyl-amino]-3-methyl-2-butensäureethylester.

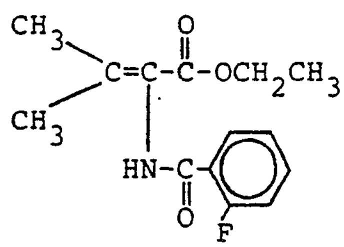

$C_{14}H_{16}NO_3F$(265,285)

| | %C | %H | %N |
|---|---|---|---|
| Berechnet: | 63,38 | 6,08 | 5,28 |
| Gefunden: | 63,06 | 6,40 | 5,23 |

Schmelzpunkt: 82,5°C

$^1H$—NMR ($CDCl_3$/TMS): δ =
6,85—7,65 (m, 5H) NH; aromat.—CH;
4,2 (q, 2H) —$COOCH_2$—;
2,25 (s, 3H) $CH_3$—C=;
1,9 (s, 3H) $CH_3$—C=;
1,35 ppm (t, 3H) —$COOCH_2CH_3$.

Beispiel 47

134 mg (0,5 mMol) Ammoniumperrhenat, 8,55 g (49,9 mMol) 2-Azido-3-methyl-butansäurethylester, 3,85 g (52,6 mMol) Dimethylformamid und 50 mg (0,45 mMol) Hydrochinon wurden in 50 ml Acetonitril gelöst. Dann wurden bei Raumtemperatur unter kräftigem Rühren 8,35 g (52,6 mMol) 4-Fluorbenzoylchlorid zugegeben. Das Reaktionsgemisch wurde 14 Stunden bei Raumtemperatur gerührt und anschließend analog Beispiel 3 aufgearbeitet.

Es ergaben sich 9,3 g (70,2% der Theorie) analysenreiner 2-[(4'-Fluorphenyl)-carbonylamino]-3-methyl-2-butensäureethylester.

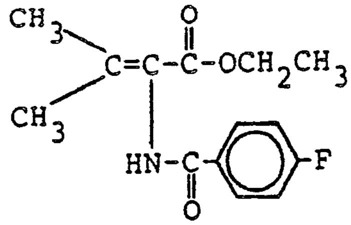

$C_{14}H_{16}NO_3F$(265,285)

| | %C | %H | %N |
|---|---|---|---|
| Berechnet: | 63,38 | 6,08 | 5,28 |
| Gefunden: | 62,94 | 6,15 | 5,11 |

Schmelzpunkt: 130 bis 132°C
$^1H$—NMR ($CDCl_3$/TMS): δ =

7,6—8,2 (m, 3H) NH; 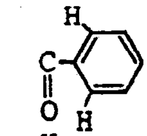

6,7—7,3 (m, 2H) 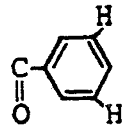

4,2 (q, 2H) —$COOCH_2$—;
2,15 (s, 3H) $CH_3$—C=;
1,85 (s, 3H) —$CH_3$—C=;
1,25 ppm (t, 3H) —$COOCH_2$—$CH_3$.

Beispiel 48

134 mg (0,5 mMol) Ammoniumperrhenat, 8,55 g (49,9 mMol) 2-Azido-3-methyl-butansäureethylester, 3,85 g (52,6 mMol) Dimethylformamid und 50 mg (0,45 mMol) Hydrochinon wurden in 50 ml Acetonitril gelöst. Dann wurden bei Raumtemperatur unter kräftigem Rühren 11,0 g (52,5 mMol) 2,4-Dichlorbenzoylchlorid zugegeben. Das Reaktionsgemisch wurde bei Raumtemperatur 14 Stunden gerührt und anschließend analog Beispiel 3 aufgearbeitet.

Es ergaben sich 9,3 g (58,9% der Theorie) analysenreiner 2-[(2',4'-Dichlorphenyl)-carbonylamino]-3-methyl-2-butensäureethylester.

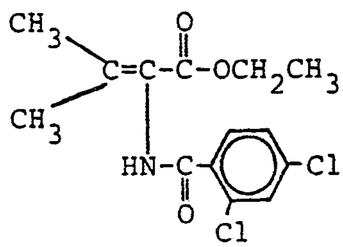

$C_{14}H_{15}NO_2Cl_2$(316,18)

| | %C | %H | %N | %Cl |
|---|---|---|---|---|
| Berechnet: | 53,18 | 4,78 | 4,43 | 22,43 |
| Gefunden: | 53,22 | 4,80 | 4,41 | 20,73 |

Schmelzpunkt: 128,5 bis 130°C
$^1H$—NMR ($CDCl_3$/TMS): δ =
7,1—7,7 (m, 4H) NH; aromat.—CH;
4,2 (q, 2H) —$COOCH_2$—;
2,2 (s, 3H) $CH_3$—C=;
1,95 (s, 3H) $CH_3$—C=;
1,3 ppm (t, 3H) $COOCH_2$—$CH_3$.

Beispiel 49

134 mg (0,5 mMol) Ammoniumperrhenat, 8,55 g (49,9 mMol) 2-Azido-3-methyl-butansäureethylester,

3,85 g (52,6 mMol) Dimethylformamid und 50 mg (0,45 mMol) Hydrochinon wurden in 50 ml Acetonitril vorgelegt. Dann wurden bei Raumtemperatur unter kräftigem Rühren 11,0 g (52,5 mMol) 3,4-Dichlorbenzoylchlorid zugegeben. Das Reaktionsgemisch wurde bei Raumtemperatur 15 Stunden gerührt und anschließend analog Beispiel 3 aufgearbeitet.

Es ergaben sich 10,5 g (66,5% der Theorie) analysenreiner 2-[(3',4'-Dichlorphenyl)-carbonyl]-3-methyl-2-butensäureethylester.

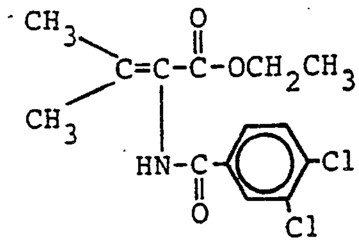

$C_{14}H_{15}NO_3Cl_2$(316,18)

| | %C | %H | %N | %Cl |
|---|---|---|---|---|
| Berechnet: | 53,18 | 4,78 | 4,43 | 22,43 |
| Gefunden: | 53,40 | 4,86 | 4,36 | 22,11 |

Schmelzpunkt: 115 bis 120°C

$^1$H—NMR ($CDCl_3$/TMS): δ =

7,2—8,2 (m, 4H) NH; aromat.—CH;
4,2 (q, 2H) —$COOCH_2$—;
2,15 (s, 3H) $CH_3$—C=;
1,9 (s, 3H) $CH_3$—C=;
1,25 ppm (t, 3H) —$COOCH_2CH_3$.

Beispiel 50

134 mg (0,5 mMol) Ammoniumperrhenat, 8,55 g (49,9 mMol) 2-Azido-3-methyl-butansäureethylester, 3,85 g (52,6 mMol) Dimethylformamid und 50 mg (0,45 mMol) Hydrochinon wurden in 50 ml Acetonitril vorgelegt. Dann wurden bei Raumtemperatur unter kräftigem Rühren 11,0 g (52,5 mMol) 3,5-Dichlorbenzoylchlorid zugegeben. Das Reaktionsgemisch wurde 15 Stunden bei Raumtemperatur gerührt. Das ausgefallene Rohprodukt wurde abgesaugt und mit Wasser Chlorid-frei gewaschen. Es ergaben sich 12,3 g (77,9% der Theorie) 2-[(3',5'-Dichlorphenyl)-carbonylamino]-3-methyl-2-butensäureethylester.

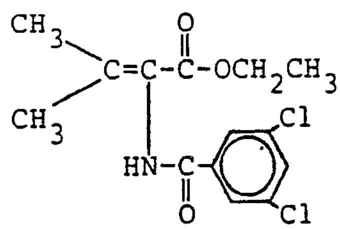

$C_{14}H_{15}NO_3Cl_2$(316,18)

| | %C | %H | %N | %Cl |
|---|---|---|---|---|
| Berechnet: | 53,18 | 4,78 | 4,43 | 22,43 |
| Gefunden: | 50,44 | 4,88 | 3,91 | 21,12 |

Schmelzpunkt: 171°C

$^1$H—NMR ($CDCl_3$/TMS): δ =

7,15—7,85 (m, 4H) NH; aromat.—CH;
4,2 (q, 2H) —$COOCH_2$—;
2,2 (s, 3H) $CH_3$—C=;
1,9 (s, 3H) $CH_3$—C=;
1,25 ppm (t, 3H) —$COOCH_2$—$CH_3$.

Beispiel 51

134 mg (0,5 mMol) Ammoniumperrhenat, 8,55 g (49,9 mMol) 2-Azido-3-methyl-butansäureethylester, 3,85 g (52,6 mMol) Dimethylformamid und 50 mg (0,45 mMol) Hydrochinon wurden in 50 ml Acetonitril vorgelegt. Dann wurden bei Raumtemperatur unter kräftigem Rühren 12,6 g (52,6 mMol) 2,6-Dichlorphenoxy-acetylchlorid zugegeben. Das Reaktionsgemisch wurde 14 Stunden bei Raumtemperatur gerührt. Das ausgefallene Rohprodukt wurde abgesaugt und mit Wasser Chlorid-frei gewaschen. Es

ergaben sich 14,4 g (87,4% der Theorie) analysenreiner 2-[(2',6'-Dichlorphenoxy)-acetylamino]-3-methyl-2-butensäureethylester.

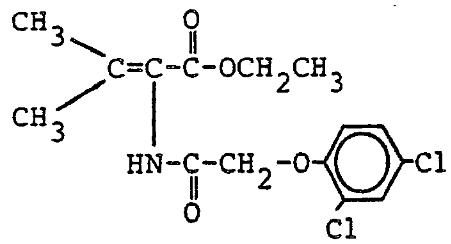

$C_{15}H_{17}NO_3Cl_3$(330,21)

| | %C | %H | %N | %Cl |
|---|---|---|---|---|
| Berechnet: | 54,56 | 5,19 | 4,24 | 21,47 |
| Gefunden: | 50,24 | 5,12 | 4,12 | 20,30 |

Schmelzpunkt: 124 bis 125°C
$^1H$—NMR ($CDCl_3$/TMS): δ =
7,95 (s breit, 1H) NH;
6,65—7,5 (m, 2H) aromat. —CH;
4,6 (s, 2H) $COCH_2$—O—;
4,2 (q, 2H) $COOCH_2$;
2,2 (s, 3H) $CH_2$—C=;
1,9 (s, 3H) $CH_3$—C=;
1,25 ppm (t, 3H) $COOCH_2CH_3$.

Beispiel 52

134 mg (0,5 mMol) Ammoniumperrhenat, 8,55 g (49,9 mMol) 2-Azido-3-methyl-butansäureethylester, 3,85 g (52,6 mMol) Dimethylformamid und 50 mg (0,45 mMol) Hydrochinon wurden in 50 ml Acetonitril vorgelegt. Dann wurden bei Raumtemperatur 11,3 g (52,5 mMol) 4,5-Dichlorthiophen-2-carbonsäurechlorid zugegeben. Das Reaktionsgemisch wurde 15 Stunden bei Raumtemperatur gerührt, wobei eine maximale Reaktionsexotherme von 45°C erreicht wurde. Die Aufarbeitung erfolgte analog Beispiel 3.

Es ergaben sich 11,6 g (72,1% der Theorie) analysenreiner 2-[2'-(4,5-Dichlorthienyl)-carbonylamino]-3-methyl-2-butensäureethylester.

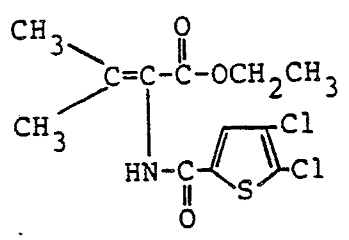

$C_{12}H_{12}NO_3Cl_2S$(322,21)

| | %C | %H | %N | %S | %Cl |
|---|---|---|---|---|---|
| Berechnet: | 44,73 | 4,07 | 4,35 | 9,49 | 22,00 |
| Gefunden: | 43,16 | 4,20 | 3,60 | 9,49 | 21,65 |

Schmelzpunkt: 95 bis 98°C
$^1H$—NMR ($CDCl_3$/TMS): δ =
8,45 (s, 1H) NH;
7,55 (s, 1H) 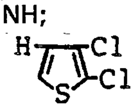
4,2 (q, 2H) —$COOCH_2$—;
2,15 (s, 3H) $CH_3$—C=;
1,9 (s, 3H) $CH_3$—C=;
1,3 ppm (t, 3H) —$COOCH_2$—$CH_3$.

Beispiel 53

134 mg (0,5 mMol) Ammoniumperrhenat, 8,55 g (49,9 mMol) 2-Azido-3-methyl-butansäureethylester, 3,85 g (52,6 mMol) Dimethylformamid und 50 mg (0,45 mMol) Hydrochinon wurden in 50 ml Acetonitril vorgelegt. Dann wurden bei Raumtemperatur unter kräftigem Rühren 7,1 g (52,7 mMol) 2,2-Dimethylbutansäurechlorid zugegeben. Das Reaktionsgemisch wurde 35 Stunden bei Raumtemperatur gerührt und anschließend analog Beispiel 3 aufgearbeitet. Das Rohprodukt wurde zur Reinigung im Vakuum (0,53

mbar) bei 110 bis 150°C im Kugelrohr destilliert. Das so erhaltene Öl wurde mit n-Pentan zur Kristallisation gebracht.

Es ergaben sich 8,8 g (73,1% der Theorie) analysenreiner 2-[(1',1'-Dimethylpropyl)-carbonylamino]-3-methyl-2-butensäureethylester.

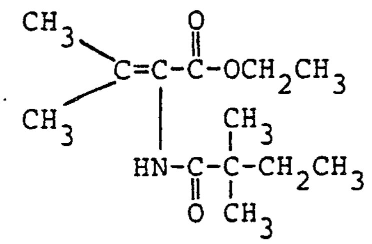

$C_{12}H_{23}NO_3$(241,33)

| | %C | %H | %N |
|---|---|---|---|
| Berechnet: | 64,70 | 9.61 | 5,80 |
| Gefunden: | 64,81 | 9,89 | 5,67 |

Schmelzpunkt: 100 bis 105°C
$^1H$—NMR ($CDCl_3$/TMS): δ =
6,85 (s breit, 1H) NH;
4,15 (q, 1H) —$COOCH_2$;
2,15 (s, 3H) $CH_3$—C=;
1,8 (s, 3H) $CH_3$—C=;
0,65—1,85 ppm (m, 14H) C—($CH_3$—$CH_2CH_3$; O—$CH_2CH_3$.

Beispiel 54

13,7 mg (0,05 mMol) Natriumperrhenat, 926 mg (5 mMol) 2-Azido-3-methyl-butansäureisopropylester und 938,8 mg (5,25 mMol) Dimethylformamid wurden in 2,5 ml Acetonitril gelöst. Dann wurden bei Raumtemperatur unter kräftigem Rühren 592,9 mg (5,25 mMol) Chloracetylchlorid zugegeben. Das Reaktionsgemisch wurde 7 Stunden bei Raumtemperatur gerührt und anschließend analog Beispiel 1 aufgearbeitet.

Es ergaben sich 1,0657 g (91,2% der Theorie) analysenreiner 2-Chlormethylcarboxamido-3-methyl-2-butensäureisopropylester.

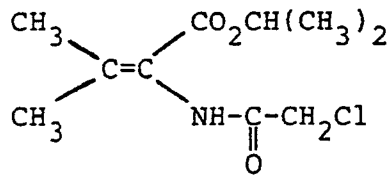

$C_{10}H_{16}ClNO_3$(233,695)

| | %C | %H | %N | %Cl |
|---|---|---|---|---|
| Berechnet: | 51,40 | 6,90 | 5,99 | 15,17 |
| Gefunden: | 51,41 | 6,97 | 5,77 | 15,16 |

Schmelzpunkt: 126°C
$^1H$—NMR ($CDCl_3$): δ =
7,9 (s, 1H) NH;
5,13 (septett, 1H) O—$CH(CH_3)_2$;
4,15 (s, 2H) $CH_2Cl$;
2,2 (s, 3H)
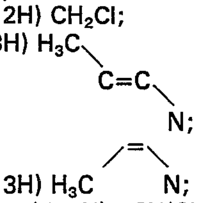
1,88 (s, 3H)
1,29 ppm (d, 6H)—$CH(CH_3)_2$.
IR $\nu_{NH}$ = 3265 $cm^{-1}$,
$\nu_{CO}$ = 1712 $cm^{-1}$, 1663 $cm^{-1}$.

Beispiel 55

13,7 mg (0,05 mMol) Natriumperrhenat, 1,17 g (5 mMol) 2-Azido-3-methyl-butansäurebenzylester und 383,8 mg (5,25 mMol) Dimethylformamid wurden in 1,5 ml Acetonitril gelöst. Dann wurden bei

Raumtemperatur unter kräftigem Rühren 592,9 mg (5,25 mMol) Chloracetylchlorid zugegeben. Das Reaktionsgemisch wurde 15 Stunden bei Raumtemperatur gerührt und anschließend analog Beispiel 1 aufgearbeitet.

Es ergaben sich 1,32 g (93,7% der Theorie) analysenreiner 2-Chlormethylcarboxamido-3-methyl-2-butensäurebenzylester.

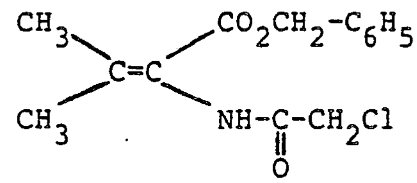

$C_{14}H_{16}ClNO_3$(281,739)

| | %C | %H | %N | %Cl |
|---|---|---|---|---|
| Berechnet: | 59,68 | 5,72 | 4,97 | 12,58 |
| Gefunden: | 59,75 | 5,64 | 4,77 | 12,60 |

Schmelzpunkt: 122 bis 122,5°C

$^1$H—NMR (CDCl$_3$): δ =

7,76 (s, 1H) NH;

7,4 (s, 5H) aromat.—CH=;

5,25 (s, 2H) —O—CH$_2$—;

4,13 (s, 2H) —CH$_2$Cl;

2,24 (d, 3H) 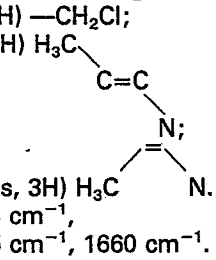

1,9 ppm (s, 3H)

IR $\nu_{NH}$ = 3255 cm$^{-1}$,

$\nu_{CO}$ = 1705 cm$^{-1}$, 1660 cm$^{-1}$.

Beispiel 56

13,7 mg (0,05 mMol) Natriumperrhenat, 1,0961 g (5 mMol) 2-Azido-3-methyl-butansäurephenylester und 383,8 mg (5,25 mMol) Dimethylformamid wurden in 1,5 ml Acetonitril gelöst. Dann wurden bei Raumtemperatur unter kräftigem Rühren 592,9 mg (5,25 mMol) Chloracetylchlorid zugegeben. Das Reaktionsgemisch wurde 15 Stunden bei Raumtemperatur gerührt und anschließend analog Beispiel 1 aufgearbeitet.

Es ergaben sich 1,24 g (92,6% der Theorie) analysenreiner 2-Chlormethylcarboxamido-3-methyl-2-butensäurephenylester.

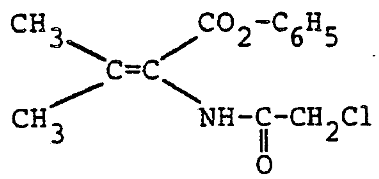

$C_{13}H_{14}ClNO_3$(267,712)

| | %C | %H | %N | %Cl |
|---|---|---|---|---|
| Berechnet: | 58,32 | 5,27 | 5,23 | 13,24 |
| Gefunden: | 58,34 | 5,20 | 4,96 | 13,42 |

Schmelzpunkt: 126°C

$^1$H—NMR (CDCl$_3$): δ =

7,91 (s, 1H) NH;

7,63—7,1 (m, 5H) aromat.—CH=;

4,2 (s, 2H) CH$_2$Cl;

2,29 (d, 3H) 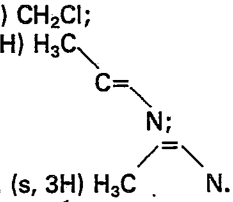

1,98 ppm (s, 3H)

IR $\nu_{NH}$ = 3280 cm$^{-1}$,

$\nu_{CO}$ = 1724 cm$^{-1}$, 1653 cm$^{-1}$.

Beispiel 57

27,3 mg (0,1 mMol) Natriumperrhenat und 1,57 g (10 mMol) 2-Azido-3-methyl-butansäuremethylester wurden bei 80°C in 5 ml Essigsäureethylester vorgelegt. Dann wurden innerhalb von 45 Minuten unter kräftigem Rühren 1,55 g (10,5 mMol) Dichloracetylchlorid zugetropft. Das Reaktionsgemisch wurde unter kräftigem Rühren insgesamt 3 Stunden auf 80°C gehalten und anschließend analog Beispiel 1 aufgearbeitet.

Es ergaben sich 2,27 g (94,7% der Theorie) analysenreiner 2-Dichlormethylcarboxamido-3-methyl-2-butensäuremethylester.

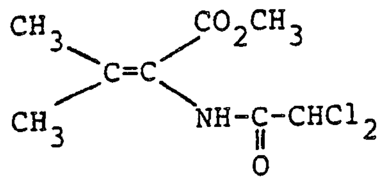

$C_8H_{11}Cl_2NO_3$(240,086)

| | %C | %H | %N | %Cl |
|---|---|---|---|---|
| Berechnet: | 40,02 | 4,62 | 5,83 | 29,53 |
| Gefunden: | 39,92 | 4,51 | 5,78 | 29,72 |

Schmelzpunkt: 118°C

$^1$H—NMR ($CDCl_3$): δ =
7,75 (s, 1H) NH;
6,05 (s, 1H) $CHCl_2$;
3,79 (s, 3H) $OCH_3$;
2,26 (d, 3H) $H_3C$

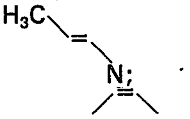

N;

1,9 ppm (s, 3H) $H_3C$ N.

IR $\nu_{NH}$ = 3265 $cm^{-1}$,
$\nu_{CO}$ = 1722 $cm^{-1}$, 1670 $cm^{-1}$.

Beispiel 58

27,3 mg (0,1 mMol) Natriumperrhenat und 1,57 g (10 mMol) 2-Azido-3-methyl-butansäuremethylester wurden bei 80°C in 5 ml Essigsäureethylester vorgelegt. Dann wurden innerhalb von 15 Minuten unter kräftigem Rühren 1,48 g (10,5 mMol) Benzoylchlorid zugetropft. Das Reaktionsgemisch wurde unter kräftigem Rühren ingesamt 5 Stunden auf 80°C gehalten und anschließend analog Beispiel 1 aufgearbeitet.

Es ergaben sich 2,0877 g (89,5% der Theorie) analysenreiner 2-Phenylcarboxamido-3-methyl-2-butensäuremethylester.

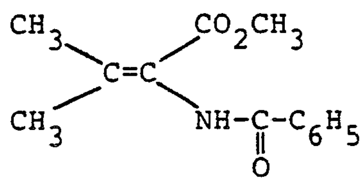

$C_{13}H_{15}NO_3$(233,267)

| | %C | %H | %N |
|---|---|---|---|
| Berechnet: | 66,94 | 6,48 | 6,00 |
| Gefunden: | 66,94 | 6,59 | 6.01 |

Schmelzpunkt: 132°C

$^1$H—NMR ($CDCl_3$): δ =
7,91—7,29 (m, 5H) aromat.—CH=;
3,72 (s, 3H) $OCH_3$;
2,18 (d, 3H) $CH_3$

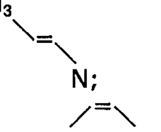

N;

1,89 ppm (s, 3H) $CH_3$ N.

IR $\nu_{NH}$ = 3280 $cm^{-1}$,
$\nu_{CO}$ = 1725 $cm^{-1}$, 1643 $cm^{-1}$.

Beispiel 59

27,3 (0,1 mMol) Natriumperrhenat und 1,57 g (10 mMol) 2-Azido-3-methyl-butensäuremethylester wurden bei 80°C in 5 ml Essigsäureethylester vorgelegt. Dann wurden innerhalb von 30 Minuten unter kräftigem Rühren 1,10 g (10,5 mMol) Cyclopropancarbonsäurechlorid, gelöst in 5 ml Essigsäureethylester, zugetropft. Das Reaktionsgemisch wurde unter kräftigem Rühren insgesamt 4 Stunden auf 80°C gehalten und anschließend analog Beispiel 1 aufgearbeitet.

Es ergaben sich 1,74 g (88,3% der Theorie) analysenreiner 2-Cyclopropylcarboxamido-3-methyl-2-butensäuremethylester.

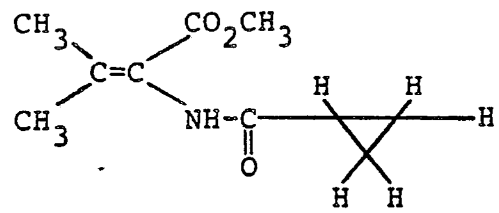

$C_{10}H_{15}NO_3$(197,234)

| | %C | %H | %N |
|---|---|---|---|
| Berechnet: | 60,90 | 7,67 | 7,10 |
| Gefunden: | 60,65 | 7,53 | 7,05 |

Schmelzpunkt: 150°C

$^1H$—NMR (CDCl₃): δ =

7,13 (s, 1H) NH;

3,74 (s, 3H) $OCH_3$;

2,11 (s, 3H)

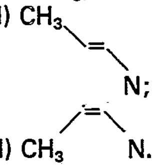

1,84 (s, 3H)

1,68—0,6 ppm (m, 5H)

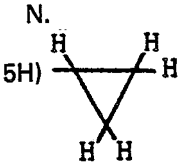

IR $\nu_{NH}$ = 3270 $cm^{-1}$,

$\nu_{CO}$ = 1719 $cm^{-1}$, 1644 $cm^{-1}$.

Beispiel 60

27,3 mg (0,1 mMol) Natrimperrhenat und 1,57 g (10 mMol) 2-Azido-3-methyl-butansäuremethylester wurden bei 80°C in 5 ml Essigsäureethylester vorgelegt. Dann wurden innerhalb von 30 Minuten unter kräftigem Rühren 1,25 g (10,5 mMol) 1-Methyl-cyclopropancarbonsäurechlorid, gelöst in 5 ml Essigsäureethylester, zugetropft. Das Reaktionsgemisch wurde unter kräftigem Rühren insgesamt 4 Stunden auf 80°C gehalten und anschließend analog Beispiel 1 aufgearbeitet.

Es ergaben sich 1,95 (92,4% der Theorie) analysenreiner 2-[(1-Methylcyclopropyl)-carboxamido]-2-methyl-2-butensäuremethylester.

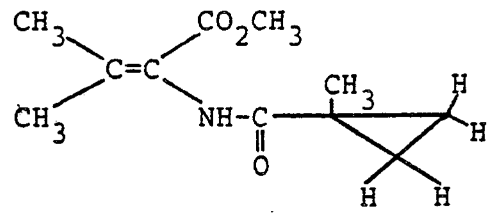

$C_{11}H_{17}NO_3$(211,261)

| | %C | %H | %N |
|---|---|---|---|
| Berechnet: | 62,54 | 8,11 | 6,63 |
| Gefunden: | 62,34 | 7,97 | 6,71 |

Schmelzpunkt: 73,5°C
$^1H$—NMR ($CDCl_3$): δ =
6,97 (s, 1H) NH;
3,77 (s, 3H) $OCH_3$;
2,16 (s, 3H) $CH_3$
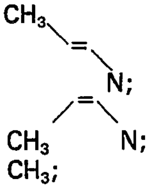
1,84 (s, 3H) $CH_3$
1,43 (s, 3H) $CH_3$;
2,0—0,5 ppm (m, 4H) 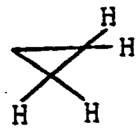

IR $\nu_{NH}$ = 3225 $cm^{-1}$,
$\nu_{CO}$ = 1717 $cm^{-1}$, 1645 $cm^{-1}$.

Beispiel 61

27,3 mg (0,1 mMol) Natriumperrhenat und 1,57 g (10 mMol) 2-Azido-3-methylbutansäuremethylester wurden bei 80°C in 5 ml Essigsäureethylester vorgelegt. Dann wurden innerhalb von 30 Minuten unter kräftigem Rühren 1,97 g (10,5 mMol) 2,2-Dichlor-1-methyl-cyclopropan-carbonsäurechlorid, gelöst in 5 ml Essigsäureethylester, zugetropft. Das Reaktionsgemisch wurde unter kräftigem Rühren insgesamt 4 Stunden auf 80°C gehalten und anschließend analog Beispiel 1 aufgearbeitet. Es ergaben sich 2,61 g (93,1% der Theorie) analysenriener 2-[(2',2'-Dichlor-1'-methyl)-cyclopropylcarboxamido)-3-methyl-2-butensäuremethylester.

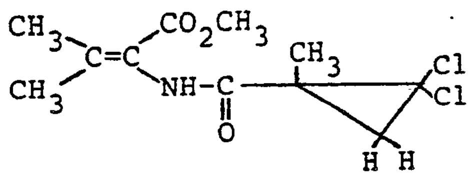

$C_{11}H_{15}Cl_2NO_3$(280,151)

| | %C | %H | %N | %Cl |
|---|---|---|---|---|
| Berechnet: | 47,16 | 5,40 | 5,00 | 25,31 |
| Gefunden: | 47,05 | 5,45 | 4,88 | 25,35 |

Schmelzpunkt: 104°C
$^1H$—NMR ($CDCl_3$): δ =
7,31 (s, 1H) NH;
3,71 (s, 3H) $OCH_3$;
2,25 (d, 1H) 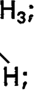
2,18 (s, 3H) $CH_3$
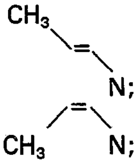
1,86 (s, 3H) $CH_3$
1,66 (s, 3H) $CH_3$;
1,39 ppm (d, 1H) 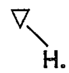

IR $\nu_{NH}$ = 3280 $cm^{-1}$,
$\nu_{CO}$ = 1721 $cm^{-1}$, 1650 $cm^{-1}$.

Beispiel 62

27,3 mg (0,1 mMol) Natriumperrhenat und 1,57 g (10 mMol) 2-Azido-3-methylbutansäuremethylester wurden bei 80°C in 5 ml Essigsäureethylester vorgelegt. Dann wurden innerhalb von 30 Minuten unter kräftigem Rühren 2,39 g (10,5 mMol) 2-(2',2'-Dichlorethenyl)-3,3-dimethyl-cyclopropancarbonsäurechlorid, gelöst in 5 ml Essigsäureethylester, zugetropft. Das Reaktionsgemisch wurde unter kräftigem Rühren insgesamt 4 Stunden auf 80°C gehalten und anschließend analog Beispiel 1 aufgearbeitet.

Es ergaben sich 3,08 g (96,3% der Theorie) analysenreiner 2-[2'-(2,2-Dichlorethenyl)-3',3'-dimethyl]-cyclopropylcarboxamido-3-methyl-2-butensäuremethylester.

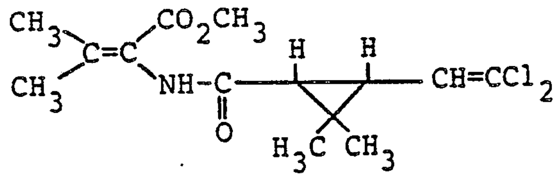

$C_{14}H_{19}Cl_2NO_3$(320,216)

| | %C | %H | %N | %Cl |
|---|---|---|---|---|
| Berechnet: | 52,51 | 5,98 | 4,37 | 22,14 |
| Gefunden: | 52,29 | 6,13 | 4,25 | 22,40 |

Schmelzpunkt: 151,5 bis 152,5°C
$^1H$—NMR ($CDCl_3$): δ =
6,69 (s, 1H) NH;
6,46 (d, 1H) —CH=$CCl_2$;
3,76 (s, 3H) $OCH_3$;
2,16 (s, 3H) 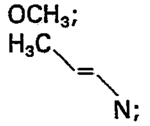
1,85 (s, 3H) 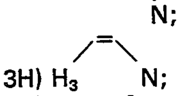
1,29 (s, 3H)
1,27 ppm (s, 3H) 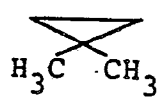

IR $\nu_{NH}$ = 3345 $cm^{-1}$,
$\nu_{CO}$ = 1770 $cm^{-1}$, 1665 $cm^{-1}$.

Beispiel 63

246 mg (0,5 mMol) Tetra-n-butyl-ammoniumperrhenat, 7,85 g (50 mMol) 2-Azido-3-methylbutansäuremethylester, 5,2 g (52,5 mMol) N-Methylpyrrolidin und 50 mg (0,45 mMol) Hydrochinon wurden in 50 ml Acetonitril gelöst. Dann wurden bei Raumtemperatur unter kräftigem Rühren 9,75 g (52,5 mMol) 4-Nitrobenzoylchlorid zugegeben. Das Reaktionsgemisch wurde 14 Stunden bei Raumtemperatur gerührt und anschließend analog Beispiel 3 aufgearbeitet.

Es ergaben sich 8,9 g (64,0% der Theorie) analysenreiner 2-[(4'-Nitrophenyl)-carbonylamino]-3-methyl-2-butensäuremethylester.

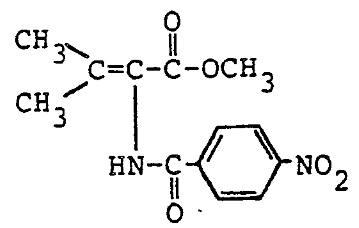

$C_{13}H_{14}N_2O_5$(278,27)

| | %C | %H | %N |
|---|---|---|---|
| Berechnet: | 56,11 | 5,07 | 10,07 |
| Gefunden: | 55,84 | 5,13 | 9,21 |

Schmelzpunkt: 122°C
$^1H$—NMR ($CDCl_3$/TMS): δ =
8,4—7,6 (m, 5H) NH; aromat. CH=;
3,75 (s, 3H) $COOCH_3$;
2,15 (s, 3H) $CH_3$—;
1,9 ppm (s, 3H) $CH_3$—.

Beispiel 64

246 mg (0,5 mMol) Tetra-n-butyl-ammoniumperrhenat, 7,85 g (50 mMol) 2-Azido-3-methylbutansäuremethylester, 5,2 g (52,5 mMol) N-Methylpyrrolidon und 50 mg (0,45 mMol) Hydrochinon wurden in 50 ml Acetonitril gelöst. Dann wurden bei Raumtemperatur unter kräftigem Rühren 11,05 g (52,5 mMol) 2,6-Dichlorisonicotinsäurechlorid zugegeben. Der Ansatz reagierte innerhalb 15 Minuten exotherm ab, wobei das Rohprodukt ausfiel. Dieses wurde abgesaugt und mit Wasser Chlorid-frei gewaschen.

Es ergaben sich 8,9 g (58,7% der Theorie) analysenreiner 2-[(2',6'-Dichlorisonicotinoyl)-amino]-3-methyl-2-butensäuremethylester.

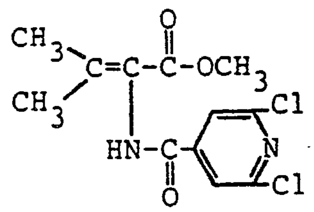

$C_{12}H_{12}N_2O_3Cl_2$(303,15)

| | %C | %H | %N | %Cl |
|---|---|---|---|---|
| Berechnet: | 47,54 | 4,00 | 9,24 | 23,40 |
| Gefunden: | 47,50 | 3,84 | 8,39 | 22,69 |

Schmelzpunkt: 186 bis 187°C
$^1$H—NMR (DMSO—$\delta_6$/TMS): δ =
10,1 (s breit, 1H) NH;
7,95 (s, 2H) aromat. CH;
3,6 (s, 3H) —$COOCH_3$;
2,1 (s, 3H) $CH_3$—;
1,85 ppm (s, 3H) $CH_3$—.

Beispiel 65

246 mg (0,5 Mol) Tetra-n-butyl-ammoniumperrhenat 9,25 g (50 mMol) 2-Azido-butansäure-n-butylester, 3,85 g (52,6 mMol) Dimethylformamid und 50 mg (0,45 mMol) Hydrochinon wurden in 50 ml Acetonitril gelöst. Dann wurden bei Raumtemperatur unter kräftigem Rühren 11,05 g (52,5 mMol) 2,6-Dichlorisonicotinsäurechlorid zugegeben. Der Ansatz reagierte innerhalb 15 Minuten exotherm ab. Die Aufarbeitung erfolgte analog Beispiel 3.

Es ergaben sich 11,8 g (71,25% der Theorie) analysenreiner 2-[(2',6'-Dichlorisonicotinoyl)-amino]-2-butensäure-n-butylester.

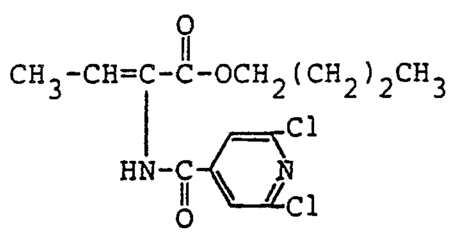

$C_{14}H_{16}N_2O_3Cl_2$(331,20)

| | %C | %H | %N | %Cl |
|---|---|---|---|---|
| Berechnet: | 50,77 | 4,87 | 8,46 | 21,40 |
| Gefunden: | 50,82 | 4,65 | 8.08 | 20,01 |

Schmelzpunkt: 123 bis 124°C
$^1$H—NMR ($CDCl_3$/TMS): δ =
8.05 (s breit, 1H) NH;
7,65 (s, 2H) aromat. CH;
6,9 (q, 1H) —CH=;
4,15 (t, 2H) —$COOCH_2$—;
1,75 (d, 3H) $CH_3$—;
0,55—2,0 ppm (m, 7H) —$CH_2CH_2$—$CH_3$.

Beispiel 66

27,3 mg (0,1 mMol) Natriumperrhenat und 2,05 g (10 mMol) 2-Azido-3-phenyl-propansäuremethylester wurden bei 80°C in 5 ml Essigsäureethylester vorgelegt. Dann wurden innerhalb von 40 Minuten unter kräftigem Rühren 1,95 g (10,5 mMol) 2-Brom-butansäurechlorid, gelöst in 5 ml Essigsäureethylester, zugetropft. Das Reaktionsgemisch wurde unter kräftigem Rühren insgesamt 3 Stunden auf 80°C gehalten und anschließend analog Beispiel 1 aufgearbeitet.

Es ergaben sich 2,23 g (68,2% der Theorie) analysenreiner 2-[(2'-Brompropyl)-carboxamido]-3-phenyl-propensäuremethylester.

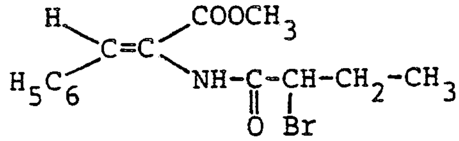

$C_{14}H_{16}BrNo_3$(326,195)

| | %C | %H | %N | %Br |
|---|---|---|---|---|
| Berechnet: | 51,55 | 4,94 | 4,29 | 24,50 |
| Gefunden: | 51,49 | 4,87 | 4,30 | 24,27 |

Schmelzpunkt: 115°C

$^1$H—NMR (CDCl$_3$): δ =
7,80 (s, 1H) NH;
7,63—7.25 (m, 5H) aromat. —CH=;
4,38 (t, 1H) —CHBr—;
3,86 (s, 3H) $OCH_3$;
2,4—1,95 (m, 2H) —$CH_2$—$CH_3$;
1,11 ppm (t, 3H) —$CH_2$—$CH_3$.

IR $\nu_{NH}$ = 3210 cm$^{-1}$,
$\nu_{CO}$ = 1715 cm$^{-1}$, 1661 cm$^{-1}$.

Beispiel 67

2,46 mg (0,5 mMol) Tetra-n-butyl-ammoniumperrhenat, 9,25 g (50 mMol) 2-Azido-butansäure-n-butylester, 5,2 g (52,5 mMol) N-Methylpyrrolidon und 50 mg (0,45 mMol) Hydrochinon wurden in 50 ml Acetonitril gelöst. Dann wurden bei Raumtemperatur unter kräftigem Rühren 10,7 g (52,3 mMol) 1-Naphthylacetylchlorid zugegeben. Das Reaktionsgemisch wurde 20 Stunden bei Raumtemperatur gerührt und anschließend analog Beispiel 3 aufgearbeitet.

Es ergaben sich 10,3 g (62,8% der Theorie) analysenreiner 2-(1'-Naphtylacetylamino)-2-butensäure-n-butylester.

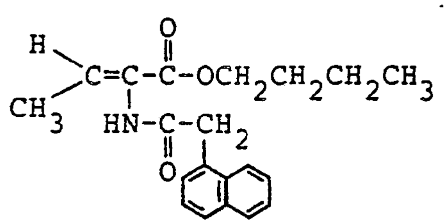

$C_{20}H_{23}NO_3$(325,41)

| | %C | %H | %N |
|---|---|---|---|
| Berechnet: | 73,82 | 7,12 | 4,30 |
| Gefunden: | 73.10 | 7,10 | 4,24 |

Schmelzpunkt: 101 bis 102°C

$^1$H—NMR (CDCl$_3$/TMS): δ =
7,1—8,2 (m, 7H) aromat. —CH;
6,8 (s breit, 1H) NH;
6,6 (q, 1H) $CH_3$—CH=;
4,05 (s, 2H) $COCH_2$—;
3,95 (t, 2H) $COOCH_2$—;
1,65 (d, 3H) $CH_3$—C=;
0,5—1,7 ppm (m, 7H) —$CH_2CH_2CH_3$.

Beispiel 68

2,46 mg (0,5 mMol) Tetra-n-butyl-ammoniumperrhenat, 50 mg Hydrochinon und 6,85 g (52,5 mMol) Furan-2-carbonsäurechlorid wurden bei 80°C in 25 ml mit Chlorwasserstoff gesättigtem Essigsäureethylester vorgelegt. Dann wurden unter kräftigem Rühren 7,85 g (50 mMol) 2-Azido-3-methyl-butansäuremethylester, gelöst in 25 ml Essigsäureethylester, zugetropft. Nach 4 Stunden Reaktionszeit war die Gasentwicklung beendet. Die Aufarbeitung erfolgte analog Beispiel 16.

Es ergaben sich 7,4 g (66,3% der Theorie) analysenreiner 2-Furyl-carbonyl-amino-3-methyl-2-butensäuremethylester.

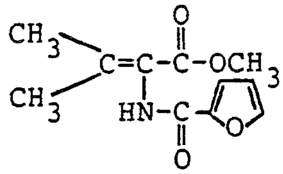

$C_{11}H_{13}NO_4$(223,23)

| | %C | %H | %N |
|---|---|---|---|
| Berechnet: | 59,19 | 5,87 | 6,27 |
| Gefunden: | 59,36 | 5,35 | 6,29 |

Schmelzpunkt: 95 bis 98°C
$^1$H—NMR ($CDCl_3$/TMS): δ =
7,55 (s breit, 1H) NH;
7,35—7,5 (m, 1H)
7,0—7,15 (m, 1H)
6,2—6,6 (m, 1H) 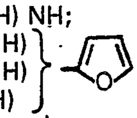
3,65 (s, 3H) $COOCH_3$;
2,2 (s, 3H) $CH_3$—C=;
1,9 ppm (s, 3H) $CH_3$—C=.

Beispiel 69

2,46 mg (0,5 mMol) Tetra-n-butyl-ammoniumperrhenat, 7,85 g (50 mMol) 2-Azido-3-methyl-butansäuremethylester, 5,2 g (52,5 mMol) N-Methylpyrrolidon und 50 mg (0,45 mMol) Hydrochinon wurden in 50 ml Acetonitril gelöst. Dann wurden bei Raumtemperatur unter kräftigem Rühren 9,55 g (52,5 mMol) Trichloracetylchlorid zugegebenen. Das Reaktionsgemisch reagierte innerhalb 20 Minuten exotherm ab. Die Aufarbeitung erfolgte analog Beispiel 3.

Es ergaben sich 7,9 g (57,6% der Theorie) analysenreiner 2-Trichlormethylcarboxamido-3-methyl-2-butensäuremethylester

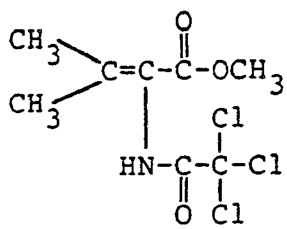

$C_8H_{10}NO_3Cl_3$(274,50)

| | %C | %H | %N | %Cl |
|---|---|---|---|---|
| Berechnet: | 35,00 | 3,67 | 5,10 | 38,75 |
| Gefunden: | 33,99 | 3,61 | 4,83 | 39,80 |

$^1$H—NMR ($CDCl_3$/TMS): δ =
7,85 (s breit, 1H) NH;
3,7 (s, 3H) $COOCH_3$;
2,25 (s, 3H) $CH_3$—;
1,85 ppm (s, 3H) $CH_3$.

Beispiel 70

2,46 mg (0,5 mMol) Tetra-n-butyl-ammoniumperrhenat, 9,25 g (50 mMol) 2-Azido-butansäure-n-butylester, 5,2 g (52,5 mMol) N-Methylpyrrolidon und 50 mg (0,45 mMol) Hydrochinon wurden in 50 ml Acetonitril gelöst. Dann wurden bei Raumtemperatur unter kräftigem Rühren 9,55 g (52,5 mMol) Trichloracetylchlorid zugegeben. Das Reaktionsgemisch wurde 16 Stunden bei Raumtemperatur gerührt und anschließend analog Beispiel 3 aufgearbeitet. Das so erhaltene Rohprodukt wurde im Hochvakuum (0,013 mbar) bei 150 bis 190°C Gerätetemperatur im Kugelrohr destilliert.

Es ergaben sich 9,5 g (62,7% der Theorie) analysenreiner 2-Trichlormethylcarboxamido-2-butensäure-n-butylester.

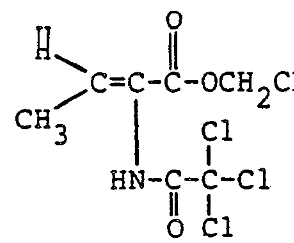

$C_{10}H_{14}NO_3Cl_3$(302,59)

| | %C | %H | %N | %Cl |
|---|---|---|---|---|
| Berechnet: | 39,69 | 4,66 | 4,63 | 35,15 |
| Gefunden: | 39,11 | 4,67 | 4,06 | 32,77 |

$^1$H—NMR ($CDCl_3$/TMS): δ =
8,05 (s breit, 1H) NH;
6,9 (q, 1H) —CH=;
4,15 (t, 2H) $COOCH_2$—;
1,75 (d, 3H) $CH_3$—C=;
0,65—1,75 ppm (m, 7H) —$CH_2CH_2CH_3$.

### Beispiel 71

134 mg (0,5 mMol) Ammoniumperrhenat, 7,85 g (50 mMol) 2-Azido-3-methyl-butansäuremethylester, 5,2 g (52,5 mMol) N-Methylpyrrolidon und 50 mg (0,45 mMol) Hydrochinon wurden in 50 ml Essigsäureethylester gelöst. Dann wurden bei Raumtemperatur unter kräftigem Rühren 8,5 g (52,2 mMol) Caprylsäurechlorid zugegeben. Der Ansatz reagierte innerhalb 10 Minuten exotherm ab. Die Aufarbeitung erfolgte analog Beispiel 16.

Es ergaben sich 9,5 g (74,4% der Theorie) analysenreiner 2-Heptylcarboxamido-3-methyl-2-butensäuremethylester.

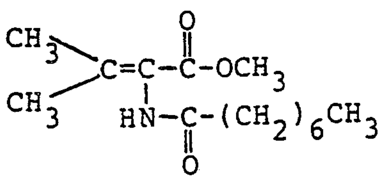

$C_{14}H_{15}O_3N$(255,36)

| | %C | %H | %N |
|---|---|---|---|
| Berechnet: | 65,85 | 9,87 | 5,48 |
| Gefunden: | 65,79 | 9,92 | 5,30 |

Schmelzpunkt: 71°C
$^1$H—NMR/$CDCl_3$/TMS: δ =
6,6 (s breit, 1H) NH;
3,65 (s, 3H) —$COOCH_3$;
1,9—2,45 (m, 2H) —$COCH_2$—;
2,1 (s, 3H) $CH_3$—C=;
1,75 (s, 3H) $CH_3$—C=;
1,25 (s, 10H) —$(CH_2)_5$=;
0,8 ppm (t, 3H) $CH_2$—$CH_3$.

### Beispiel 72

134 g (0,5 mMol) Ammoniumperrhenat, 7,85 g (50 mMol) 2-Azido-3-methyl-butansäuremethylester, 5,2 g (52,5 mMol) N-Methylpyrrolidon und 50 mg (0,45 mMol) Hydrochinon wurden in 50 ml Essigsäureethylester vorgelegt. Dann wurden bei Raumtemperatur unter kräftigem Rühren 9,0 g (52,7 mMol) Phenoxyessigsäurechlorid zugegeben. Der Ansatz reagierte innerhalb 30 Minuten exotherm ab. Die Aufarbeitung erfolgte analog Beispiel 16.

Es ergaben sich 9,0 g (68,4% der Theorie) analysenreiner 2-Phenoxyacetylamino-3-methyl-2-butensäuremethylester.

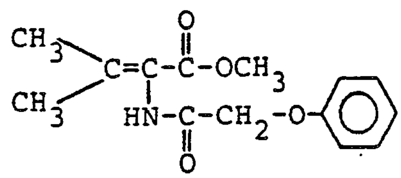

$C_{14}H_{17}O_4N$(263,3)

| | %C | %H | %N |
|---|---|---|---|
| Berechnet: | 63.86 | 6,51 | 5,32 |
| Gefunden: | 63,71 | 6,48 | 5,21 |

Schmelzpunkt: 79°C
$^1$H—NMR ($CDCl_3$/TMS): δ =
7,65 (s breit, 1H) NH;
6,65—7,55 (m, 5H) aromat. CH;
4,6 (s, 2H) —$COCH_2$—;
3,7 (s, 3H) $COOCH_3$;
2,25 (s, 3H) $CH_3$—;
1,85 ppm (s, 3H) $CH_3$—.

Beispiel 73

246 mg (0,5 mMol) Tetra-n-butylammoniumperrhenat, 15,5 g (49,7 mMol) 2-Azido-hexadecansäuremethylester, 5,2 g (15,25 mMol) N-Methylpyrrolidon und 50 mg (0,45 mMol) Hydrochinon wurden in 25 ml Essigsäureethylester und 25 ml Acetonitril gelöst. Dann wurden bei Raumtemperatur unter kräftigem Rühren 5,9 g (52,3 mMol) Chloracetylchlorid zugegeben. Der Ansatz reagierte innerhalb 30 Minuten exotherm ab. Die Aufarbeitung erfolgte analog Beispiel 3.

Es ergaben sich 11,5 g (64,3% der Theorie) analysenreiner 2-Chlormethylcarboxamido-2-hexadecensäuremethylester.

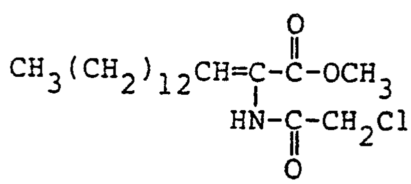

$C_{19}H_{34}NO_3Cl$(359,94)

| | %C | %H | %N | %Cl |
|---|---|---|---|---|
| Berechnet: | 63,40 | 9,52 | 3,89 | 9,85 |
| Gefunden: | 63,38 | 9,51 | 3,84 | 9,86 |

Schmelzpunkt: 74 bis 75°C
$^1$H—NMR ($CDCl_3$/TMS): δ =
7,75 (s breit, 1H) NH;
6,75 (t, 1H) —CH=;
4,1 (s, 2H) —$CH_2Cl$;
3,75 (s, 3H) —$COOCH_3$;
1,7—2,5 (m, 2H) —$CH_2$—CH=;
1,25 (s, 22H) —$(CH_2)_{11}$—;
0,85 ppm (t, 3H) $CH_3$—.

Beispiel 74

246 mg (0,5 mMol) Tetra-n-butyl-ammoniumperrhenat, 9,25 g (49,9 mMol) 2-Azido-pentansäureisopropylester, 3,85 g (52,6 mMol) Dimethylformamid und 50 mg (0,45 mMol) Hydrochinon wurden in 50 ml Acetonitril gelöst. Dann wurden bei Raumtemperatur unter kräftigem Rühren 11,8 g (52,5 mMol) 2-[4'-(2-Methylpropyl)-phenyl]-propionsäurechlorid zugegeben. Das Reaktionsgemisch wurde 15 Stunden bei Raumtemperatur gerührt und anschließend analog Beispiel 3 aufgearbeitet. Es ergaben

sich 10,0 g (58,0% der Theorie) analysenreiner 2-{2'-[4-(2'-Methylpropyl)-phenyl]-propionylamido}-2-pentensäureisopropylester.

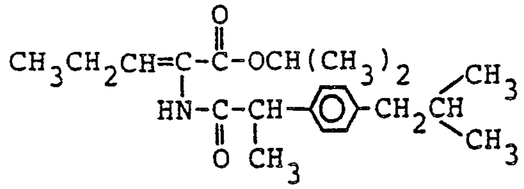

$C_{21}H_{31}NO_3$(345,48)

| | %C | %H | %N |
|---|---|---|---|
| Berechnet: | 73,00 | 9,04 | 4,05 |
| Gefunden: | 73,21 | 9,11 | 4,21 |

Schmelzpunkt: 80 bis 83°C

$^1$H—NMR ($CDCl_3$/TMS): δ =

6,9—7,3 (m, 4H) —CH= aromat.;

6,65 (s breit, 1H) NH;

6,45 (t, 1H) —$CH_2$—CH=;

4,6—5,25 (m, 1H) COOCH—;

3,65 (q, 1H) COCH—;

2,4 (d, 2H) 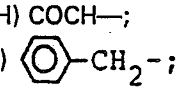

1,65—2,2 (m, 3H) —$CH_2$—CH=; —CH;

1,45 (d, 3H) CH—$CH_3$;

1,15 (d, 6H) —COOCH($CH_3$)$_2$;

0,95 (t, 3H) $CH_3$—$CH_2$;

0,85 ppm (d, 6H) —CH($CH_3$)$_2$

Beispiel 75

246 mg (0,5 mMol) Tetra-n-butyl-ammoniumperrhenat, 11,25 g (50 mMol) 2-Azido-3-cyclopentyl-propionsäureisopropylester, 3,85 g (52,6 mMol) Dimethylformamid und 50 mg (0,45 mMol) Hydrochinon wurden in 50 ml Acetonitril gelöst. Dann wurden bei Raumtemperatur 9,5 g (52,6 mMol) 3-Acetylthio-2-methylpropionsäurechlorid zugegeben. Der Ansatz reagierte innerhalb 30 Minuten exotherm ab. Die Aufarbeitung erfolgte analog zu Beispiel 3.

Es ergaben sich 9,6 g (56,2% der Theorie) analysenreiner 2-[(1'-Methyl-2'-acetylthio)-ethylcarboxamido]-3-cyclopentyl-propensäureisopropylester.

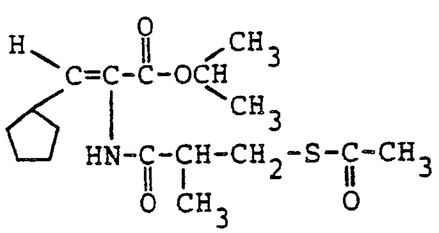

$C_{17}H_{27}NO_4S$(341,41)

| | %C | %H | %N | %S. |
|---|---|---|---|---|
| Berechnet: | 59,81 | 7,97 | 4,10 | 9,37 |
| Gefunden: | 59,55 | 8,22 | 4,12 | 8,94 |

Schmelzpunkt: 63 bis 65°C
$^1H$—NMR ($CDCl_3$/TMS): δ =
6,95 (s breit, 1H) NH;
6,6 (d, 1H) —CH=;
4,7—5,4 (m, 1H) —COOCH—;
2,15—3,5 (m, 4H) COCH—;
|
$CH_3$
2,3 (s, 3H) $COCH_3$;
1,3—2,25 (m, 9H) 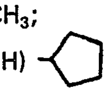
1,25 ppm (d, 6H) —$C(CH_3)_2$.

Beispiel 76

246 mg (0,5 mMol) Tetra-n-butyl-ammoniumperrhenat, 11,25 g (50 mMol) 2-Azido-3-cyclopentyl-propionsäureisopropylester, 3,85 g (52,6 mMol) Dimethylformamid und 50 mg (0,45 mMol) Hydrochinon wurden in 50 ml Acetonitril gelöst. Dann wurden bei Raumtemperatur unter kräftigem Rühren 8,5 g (52,3 mMol) Dipropylessigsäurechlorid zugegeben. Das Reaktionsgemisch wurde 16 Stunden bei Raumtemperatur gerührt und anschließend analog Beispiel 3 aufgearbeitet.

Es ergaben sich 9,2 g (56,9% der Theorie) analysenreiner 2-[(1'Propyl-butyl)-carbonylamino]-3-cyclopentyl-propensäureisopropylester.

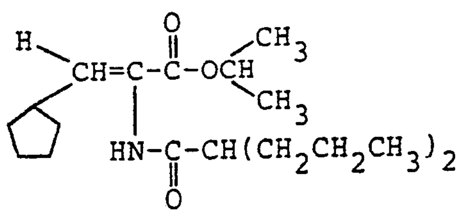

$C_{19}H_{33}NO_3$(323,48)

| | %C | %H | %N |
|---|---|---|---|
| Berechnet: | 70,55 | 10,28 | 4,33 |
| Gefunden: | 70,02 | 10,59 | 4,33 |

Schmelzpunkt: 148,2°C
$^1H$—NMR ($CDCl_3$/TMS): δ =
6,8 (s breit, 1H) NH;
6,55 (d, 1H) —CH=;
4,8—5,3 (m, 1H) COOCH—;
0,65—2,8 (m, 28H) —$CH(CH_2CH_2CH_3)_2$; 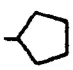
1,25 ppm (d, 6H) —$C(CH_3)_2$.

Beispiel 77

134 mg (0,5 mMol) Ammoniumperrhenat, 7,85 g (50 mMol) 2-Azido-pentansäuremethylester, 3,85 g (52,6 mMol) Dimethylformamid und 50 mg (0,45 mMol) Hydrochinon wurden in 50 ml Acetonitril gelöst. Dann wurden bei Raumtemperatur unter kräftigem Rühren 8,5 g (52,3 mMol) Dipropylessigsäurechlorid zugegeben. Das Reaktionsgemisch wurde 120 Stunden gerührt, wobei eine maximale Reaktionstemperatur von 80°C erreicht wurde. Nach Aufarbeitung analog Beispiel 3 ergaben sich 7,0 g (54,8% der Theorie) analysenreiner 2-[(1'-Propylbutyl)-carbonylamino]-2-pentensäuremethylester.

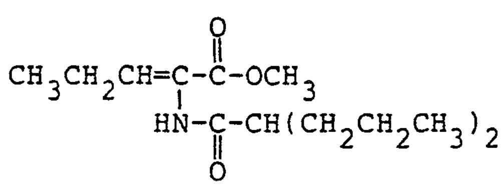

$C_{14}H_{25}NO_3$(255,36)

| | %C | %H | %N |
|---|---|---|---|
| Berechnet: | 65,85 | 9,87 | 5,48 |
| Gefunden: | 64,85 | 10,18 | 5,26 |

Schmelzpunkt: 132°C
$^1H$—NMR ($CDCl_3$/TMS): δ =
6,9 (s breit, 1H) NH;
6,65 (t, 1H) —CH=;
3,75 (s, 3H) $COOCH_3$;
1,85—2,5 (m, 2H) —$CH_2$—CH=;
0,6—1,8 ppm (m, 18H) $CH_3$—$CH_2$; —CH—($CH_2CH_2CH_3$)$_2$.

Beispiel 78

246 mg (0,5 mMol) Tetra-n-butyl-ammoniumperrhenat, 7,85 g (50 mMol) 2-Azido-3-methylbutansäuremethylester, 5,2 g (52,5 mMol) N-Methylpyrrolidon und 50 mg (0,45 mMol) Hydrochinon wurden in 50 ml Acetonitril gelöst. Dann wurden bei Raumtemperatur unter kräftigem Rühren 8,75 g (52,5 mMol) Zimtsäurechlorid zugegeben. Das Reaktionsgemisch wurde dann 14 Stunden gerührt, wobei die maximale Reaktionsexotherme 40°C betrug. Nach Aufarbeitung analog Beispiel 3 ergaben sich 9,3 g (71,7% der Theorie) analysenreiner 2-[(2′-Phenylvinyl)-carbonylamino]-3-methyl-2-butensäuremethylester.

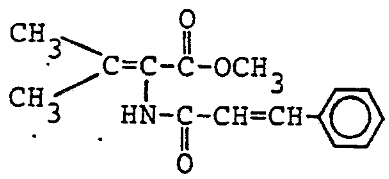

$C_{15}H_{17}NO_3$(259,31)

| | %C | %H | %N |
|---|---|---|---|
| Berechnet: | 69,48 | 6,48 | 5,40 |
| Gefunden: | 67,44 | 6,61 | 5,41 |

Schmelzpunkt: 138 bis 141°C
$^1H$—NMR ($CDCl_3$/TMS): δ =
7,1—7,6 (m, 6H) aromat. CH; NH;
7,6 (d, 1H) —CO—CH=;
6,5 (d, 1H) —C=CH—;
3,7 (s, 3H) $COOCH_3$;
2,15 (s, 3H) $CH_3$—C=;
1,85 ppm (s, 3H) $CH_3$—C=.

Beispiel 79

134 mg (0,5 mMol) Ammoniumperrhenat, 7,85 g (50 mMol) 2-Azido-pentansäuremethylester, 50 mg (0,45 mMol) Hydrochinon und 3,85 g (52,6 mMol) Dimethylformamid wurden in 50 ml Essigsäureethylester gelöst. Dann wurden bei Raumtemperatur unter kräftigem Rühren 12,0 g (52,02 mMol) 3,4,5-Trimethoxybenzoylchlorid zugetropft. Das Reaktionsgemisch wurden 240 Stunden bei Raumtemperatur gerührt. Nach Aufarbeitung analog Beispiel 16 ergaben sich 8,75 g (54,1% der Theorie) 2-[(3′,4′,5′-Trimethoxyphenyl)-carbonyl-amino]-2-pentensäuremethylester.

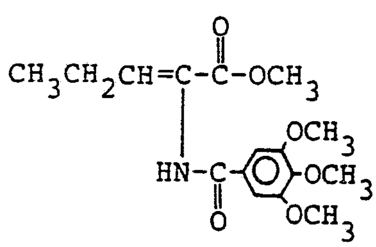

$C_{16}H_{21}NO_6$(323,35)

| | %C | %H | %N |
|---|---|---|---|
| Berechnet: | 59,43 | 6,55 | 4,33 |
| Gefunden: | 59,21 | 6,75 | 3,70 |

Schmelzpunkt: 137 bis 140°C
$^1H$—NMR ($CDCl_3$/TMS): δ =
7,65 (s breit, 1H) NH;
7,05 (s, 2H) aromat. CH=;
6,7 (t, 1H) CH=C;
3,85 (s, 3H) $COOCH_3$;
1,8—2,55 (m, 2H) —$CH_2$—;
1,05 ppm (t, 3H) $CH_3$—.

**Patentansprüche**

1. Verfahren zur Herstellung von N-Acyl-2,3-dehydroaminocarbonsäureestern der allgemeinen Formel

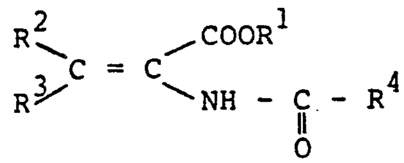

(I),

in der

$R^1$ einen geradkettigen oder verzweigten ($C_1$—$C_4$)-Alkylrest, einen Phenylrest oder einen Benzylrest,

$R^2$ Wasserstoff oder einen Methylrest,

$R^3$ Wasserstoff, einen geradkettigen oder verzweigten ($C_1$—$C_{16}$)-Alkylrest, einen ($C_3$—$C_8$)-Cycloalkylrest, einen ($C_1$—$C_6$)-Alkoxyrest, einen Phenoxyrest, einen ($C_1$—$C_6$)-Alkylmercaptorest, einen Phenylmercaptorest oder einen Methoxycarbonylmethylmercaptorest und

$R^4$ einen geradkettigen oder verzweigten unsubstituierten oder ein- oder mehrfach durch Halogen, eine Methoxygruppe oder eine Acetylthiogruppe substituierten ($C_1$—$C_{16}$)-Alkylrest oder ($C_2$—$C_{16}$)-Alkenylrest, einen unsubstituierten oder ein- oder mehrfach durch Halogen oder eine Methylgruppe substituierten ($C_3$—$C_8$)-Cycloalkylrest, einen unsubstituierten oder ein- oder mehrfach durch Halogen, eine Nitrogruppe, eine ($C_1$—$C_4$)-Alkyl- oder -Alkoxygruppe oder eine Trifluormethylgruppe substituierten Phenylrest, einen unsubstituierten oder ein- oder mehrfach durch Halogen substituierten Cinnamylrest, einen unsubstituierten oder ein- oder mehrfach durch Halogen substituierten Heteroarylrest, einen Aryl- oder Heteroarylmethylrest, einen unsubstituierten oder ein- oder mehrfach durch Halogen, eine Nitrogruppe, eine ($C_1$—$C_4$)-Alkylgruppe oder eine Trifluormethylgruppe substituierten Phenoxymethylrest oder Phenylethylrest oder einen 2-(2',2'-Dichlorethenyl)-3,3-dimethylcyclopropylrest bedeuten,

dadurch gekennzeichnet, daß man einen 2-Azidocarbonsäureester der allgemeinen Formel

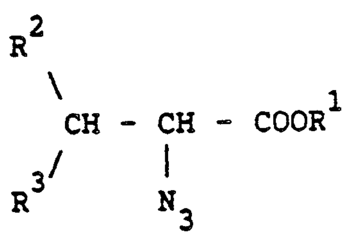

(II)

in der $R^1$, $R^2$ und $R^3$ die bereits angegebene Bedeutung haben, bei einer Temperatur zwischen 0 und 150°C in Gegenwart von Natrium-, Kalium- oder Ammoniumperrhenat oder eines substituierten Ammoniumperrhenats mit einem Carbonsäurehalogenid der allgemeinen Formel

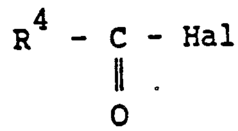

(III)

in der $R^4$ die bereits angegebene Bedeutung hat und Hal Chlor oder Brom bedeutet, zur Umsetzung bringt.

2. Verfahren nach Anspruch 1, dadurch gekennzeichnet, daß man das Perrhenat in einer Menge zwischen 0,005 und 10 Molprozent, bezogen auf die eingesetzte Menge an 2-Azidocarbonsäureester der allgemeinen Formel (II), anwendet.

3. Verfahren nach Anspruch 1 oder 2, dadurch gekennzeichnet, daß man die Umsetzung in zusätzlicher Gegenwart eines Säureamids, eines Lactams oder eines substituierten Harnstoffs vornimmt.

**Revendications**

1. Procédé pour la préparation d'esters d'acides N-acyl-2,3-déhydroaminocarboxyliques, de formule générale:

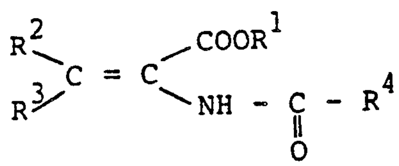

(I)

dans laquelle:

$R^1$ représente un radical alkyl en $C_1$—$C_4$ à chaîne droite ou ramifiée, un radical phényle ou un radical benzyle;

$R^2$ représente un atome d'hydrogène ou un radical méthyle;

$R^3$ représente un atome d'hydrogène ou un radical alkyle en $C_1$—$C_{16}$ à chaîne droite ou ramifiée, un radical cyclo-alkyle en $C_3$—$C_8$, un radical alcoxy en $C_1$—$C_6$, un radical phénoxy, un radical alkyl $C_1$—$C_6$-mercapto, un radical phénylmercapto ou un radical méthoxycarbonyl-méthylmercapto; et

$R^4$ représente un radical alkyle en $C_1$—$C_{16}$ ou alcényle en $C_2$—$C_{16}$, non substitué ou substitué une ou plusieurs fois par un ou des atomes d'halogène ou par un ou des groupes méthoxy ou acéthylthio, un radical cycloalkyle en $C_3$—$C_8$ non substitué ou substitué une ou plusieurs fois par un ou des atomes d'halogène ou par un ou des groupes méthyle, un radical phényle non substitué ou substitué une ou plusieurs fois par un ou des atomes d'halogène ou par un ou des groupes nitro, alkyle ou alcoxy en $C_1$—$C_4$ ou trifluorométhyle, un radical cinnamyle non substitué ou substitué une ou plusieurs fois par un ou des atomes d'halogène, un radical hétéroaryle non substitué ou substitué une ou plusieurs fois par un ou des atomes d'halogène, un radical aryle- ou hétéroarylméthyle, un radical phénoxyméthyle ou phényléthyle non substitué ou substitué une ou plusieurs fois par un ou des atomes d'halogène ou par un ou des groupes nitro, alkyle en $C_1$—$C_4$ ou trifluorométhyle, ou un radical 2-(2',2'-dichloréthényl)-3,3-diméthylcyclopropyle,

caractérisé en ce que l'on: met en réaction un ester d'acide 2-azidocarboxylique de formule générale:

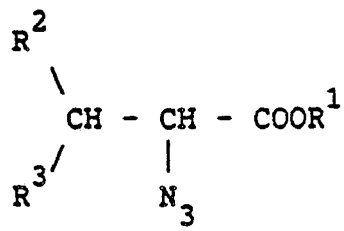

(II)

dans laquelle $R^1$, $R^2$ et $R^3$ ont les significations déjà données, à une température comprise entre 0 et 150°C, en présence de perrhénate de sodium, potassium ou ammonium ou d'un perrhénate d'ammonium substitué, avec un halogénure d'acide carboxylique de formule générale

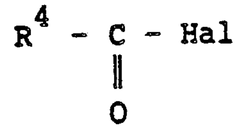

(III)

dans laquelle $R^4$ a la signification déjà donnée et Hal représente le chlore ou le brome.

2. Procédé selon la revendication 1, caractérisé en ce que l'on utilise le perrhénate en une quantité comprise entre 0,005 et 10% en mole, par rapport à la quantité utilisée d'ester d'acide 2-azidocarboxylique de formule générale (II).

3. Procédé selon la revendication 1 ou 2, caractérisé en ce que l'on effectue la réaction en présence en outre d'un amide, d'un lactame ou d'une urée substituée.

**Claims**

1. A process for the production of N-acyl-2,3-dehydroaminocarboxylic acid esters corresponding to the following general formula

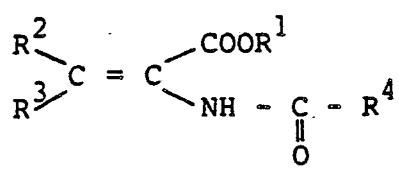

(I)

in which

$R^1$ is a linear or branched $(C_{1-4})$ alkyl radical, a phenyl radical or a benzyl radical,

$R^2$ is hydrogen or a methyl radical,

$R^3$ is hydrogen, a linear or branched $(C_{1-16})$ alkyl radical, a $(C_{3-8})$ cycloalkyl radical, a $(C_{1-6})$ alkoxy radical, a phenoxy radical, a $(C_{1-6})$ alkylmercapto radical, a phenylmercapto radical or a methoxycarbonyl methylmercapto radical and

$R^4$ is a linear or branched unsubstituted $(C_{1-16})$ alkyl radical or $(C_{2-16})$ alkenyl radical or a linear or branched $(C_{1-16})$ alkyl radical or $(C_{2-16})$ alkenyl radical mono- or polysubstituted by halogen, a methoxy group or an acetylthio group, an unsubstituted $(C_{3-8})$ cycloalkyl radical or a $(C_{3-8})$ cycloalkyl radical mono-

or polysubstituted by halogen or a methyl group, an unsubstituted phenyl radical or a phenyl radical mono- or polysubstituted by halogen, a nitro group, a $(C_{1-4})$ alkyl or alkoxy group or a trifluoromethyl group, an unsubstituted cinnamyl radical or a cinnamyl radical mono- or polysubstituted by halogen, an unsubstituted heteroaryl radical or a heteroaryl radical mono- or polysubstituted by halogen, an aryl or heteroaryl methyl radical, an unsubstituted phenoxymethyl radical or phenylethyl radical or a phenoxymethyl radical or phenylethyl radical mono- or polysubstituted by halogen, a nitro group, a $(C_{1-4})$ alkyl group or a trifluoromethyl group or a 2-(2',2'-dichloroethenyl)-3,3-dimethyl cyclopropyl radical,

characterized in that a 2-azidocarboxylic acid ester corresponding to the following general formula

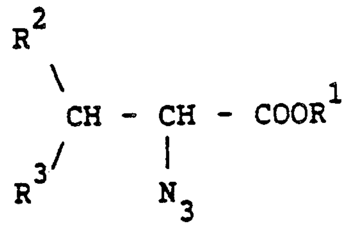

(II)

in which $R^1$, $R^2$ and $R^3$ are as already defined, is reacted with a carboxylic acid halide corresponding to the following general formula

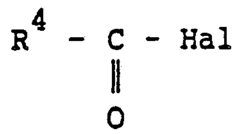

(III)

in which $R^4$ is as already defined and Hal is chlorine or bromine, in the presence of sodium, potassium or ammonium perrhenate or a substituted ammonium perrhenate at a temperature in the range from 0 to 150°C.

2. A process as claimed in claim 1, characterized in that the perrhenate is used in a quantity of from 0.005 to 10 mol-%, based on the quantity of 2-azidocarboxylic acid ester of general formula (II) used.

3. A process as claimed in claim 1 or 2, characterized in that the reaction is carried out in the additional presence of an acid amide, a lactam or a substituted urea.